# EUROPEAN PATENT SPECIFICATION

(11) **EP 2 171 568 B1**
(45) Date of publication and mention of the grant of the patent: **30.05.2018**
(21) Application number: 08770483.9
(22) Date of filing: 09.06.2008
(51) Int. Cl.: A61B 5/00, A61M 5/172, A61B 5/145, A61M 5/142, G06F 1/16, G06F 19/00

(54) **USER INTERFACE FEATURES FOR AN ELECTRONIC DEVICE**
BENUTZEROBERFLÄCHENMERKMALE FÜR EIN ELEKTRONISCHES GERÄT
FONCTIONS D'INTERFACE UTILISATEUR POUR UN DISPOSITIF ÉLECTRONIQUE

(30) Priority: 29.06.2007 US 937779 P; 29.06.2007 US 937933 P
(43) Date of publication of application: 07.04.2010
(73) Proprietor: Roche Diabetes Care GmbH, 68305 Mannheim (DE); F. Hoffmann-La Roche AG, 4070 Basel (CH)
(72) Inventor: HOLLIDAY, Rebecca, Fishers, IN 46037 (US); WERNER, Karl, 69168 Wiesloch (DE); BUSH, Jason, Fishers, IN 46038 (US); DAVIS, Stacia, McCordsville, IN 46055 (US); STEINER, Bettina, Indianapolis, IN 46220 (US); LONG, Michael, Lee, Noblesville, IN-46062 (US); GALLEY, Paul, Cumberland, IN 46229 (US); MEIERTOBERENS, Ulf, S-18273 Stocksund (SE); CELENTANO, Michael, Fishers, IN 46037 (US)
(74) Representative: Rentsch Partner AG
(86) International application number: PCT/US2008/066299
(87) International publication number: WO 2009/005958

(56) References cited:
- EP-A- 0 290 683
- WO-A-2004/099898
- WO-A2-2006/123253
- DE-A1-102004 011 135
- US-A1- 2004 008 228
- US-A1- 2004 078 416
- US-A1- 2005 039 137
- US-A1- 2005 091 577
- US-A1- 2005 162 395
- US-A1- 2006 047 192
- US-A1- 2006 262 102
- US-A1- 2007 109 325
- US-B1- 6 909 439

## Description

### FIELD OF THE INVENTION:

The present invention relates generally to user interface features for electronic devices that may include one or more on-board medical devices and that may be configured to wirelessly communicate with one or more remote medical devices.

### BACKGROUND

Electronic devices that include on-board medical devices are known. Electronic devices that are configured to wirelessly communicating with at least one medical device are also known. It is desirable with any such electronic devices to include useful user interface features.

US2006047192 A1 discloses a system for recommending insulin bolus quantities to an insulin user. The system includes a display unit and memory unit coupled to a control circuit with a user blood glucose target stored in the memory unit. The control circuit is programmed to receive the user's current blood glucose value, to determine and display via the display unit a recommended correction insulin bolus quantity if the current blood glucose value exceeds the blood glucose target, to compute a difference value as the current blood glucose value less the blood glucose target, and to produce a modified blood glucose target as a sum of the blood glucose target and the difference value for a lock-out time period if the difference value is positive. Additional correction insulin bolus quantities may be recommended during the lock-out time period if the user's current blood glucose value exceeds the modified blood glucose target. In addition, the display of the system is configured to prompt the user to enter a carbohydrate estimate corresponding to a quantity of carbohydrates that will be consumed in a subsequent meal or snack, and the control circuit is operable to compute a recommended meal compensation insulin bolus quantity based on the entered carbohydrate estimate.

US2005039137 A1 discloses a method for dynamic expansion and overlay of controls, such as input fields of a form sheet.

### SUMMARY

The present invention concerns a method of controlling a display device according to appended claim 1.

A method of controlling a display device of an electronic device may comprise displaying a data field on the display device, executing an editing process that provides for editing of the data field displayed on the display device, and magnifying the data field on the display device relative to other portions of the display device when selected for editing according to the editing process.

Magnifying the data field may comprise producing an expanded polygon about the magnified data field.

The method may further comprise displaying a plurality of data fields on the display device, and magnifying any of the plurality of data fields when selected for editing according to the editing process.

The magnified data field may be populated with a plurality of selectable choices when selected for editing according to the editing process.

The method may further comprise incrementally increasing a value displayed within the magnified data field by an increment value in response to press of a first user button of the electronic device. The method may alternatively or additionally comprise incrementally decreasing a value displayed within the magnified data field in response to press of a second user button of the electronic device. In either or both cases, the method may further comprise a fast scroll process that incrementally increases or decreases respectively the value displayed within the magnified field at a rapid rate when the corresponding first or second user button is pressed and held. Alternatively or additionally the method may further comprise a fast scroll process that increases the increment or the decrement value respectively when the corresponding first or second user button is pressed and held.

The method may further comprise automatically displaying a unit of measure of data within, or to be entered within, the magnified data field.

An electronic device may comprise electronic circuitry including a transceiver configured to wirelessly communicate with another electronic device and a housing including the transceiver contained therein. The transceiver may be configured to operate at one of a visible, infrared and ultraviolet wavelength. The housing may define a first integral window positioned over the transceiver. The first integral window may be transmissive to an operating wavelength of the transceiver such that the transceiver transmits and receives wireless information through the integral window.

The electronic circuitry may further include a display device. The housing may define a second integral window positioned over the display device such that information displayed on the display device is visible through the second integral window. The display device may be a liquid crystal display device.

The first and second integral windows may be transparent. Illustratively, he housing may be transparent, and the first and second integral windows may be formed by coating the housing with an opaque coating while masking the first and second integral windows.

An electronic device may comprise electronic circuitry including a display device configured to display information, and a housing having the electronic circuitry including the display device contained therein. The housing may define an integral, transparent window positioned over the display device such that the information displayed in the display device is visible through the integral window.

The display device may be a liquid crystal display device. The housing may be transparent, and the integral window may be formed by coating the housing with an opaque coating while masking integral window.

An electronic analyte measuring device may comprise a housing, an analyte measuring facility positioned in the housing, a display device carried by the housing and a processor. The analyte measuring facility may be configured to measure a concentration of an analyte in a liquid sample deposited on a sample carrier received in the analyte measuring facility. The processor may include a memory having instructions stored therein that are executable by the processor to automatically control the display device to display instructions to measure via the analyte measuring facility a concentration of the analyte in a subsequent liquid sample if the concentration of the analyte in the liquid sample is outside of a predetermined analyte concentration limit.

The instructions stored in the memory may further include instructions that are executable by the processor to automatically control the display device to display the instructions after a programmable time period has elapsed since measuring the concentration of the analyte in the liquid sample. The sample may be blood, the analyte may be glucose and the predetermined concentration may be a maximum blood glucose limit. The instructions stored in the memory may include instructions that are executable by the processor to automatically control the display device to display instructions to measure the concentration of the analyte in a subsequent liquid sample if the concentration of the analyte in the liquid sample is greater than the maximum blood glucose limit. The electronic analyte measuring device may further comprise at least one notification device. The maximum blood glucose limit may be a hyperglycemic limit. The instructions stored in the memory may further include instructions that are executable by the processor to activate the at least one notification device at or near the time of controlling the display device to display instructions to measure a concentration of glucose in a subsequent blood sample if the concentration of the glucose in the blood sample exceeds the hyperglycemic limit.

In an alternative embodiment, the predetermined concentration may be a minimum blood glucose limit. In this embodiment, the instructions stored in the memory may include instructions that are executable by the processor to automatically control the display device to display instructions to measure the concentration of the analyte in a subsequent liquid sample if the concentration of the analyte in the liquid sample is less than the minimum blood glucose limit. In this embodiment, the electronic analyte measuring device may further comprise at least one notification device. The minimum blood glucose limit may be a hypoglycemic limit. The instructions stored in the memory may further include instructions that are executable by the processor to activate the at least one notification device at or near the time of controlling the display device to display instructions to measure a concentration of glucose in a subsequent blood sample if the concentration of the glucose in the blood sample is less than the hypoglycemic limit.

An electronic blood glucose measuring device may comprise a housing, a blood glucose measuring facility positioned in the housing, a display device carried by the housing and a processor. The blood glucose measuring facility may be configured to measure a concentration of glucose in a blood sample deposited on a sample carrier received in the blood glucose measuring facility. The processor may execute a bolus recommendation process that recommends a bolus amount based on a number of factors including a carbohydrate value entered by a user. The processor may include a memory having instructions stored therein that are executable by the processor to automatically control the display device, after a programmable amount of time has passed since entering the carbohydrate value, to display instructions to measure blood glucose of a blood sample via the blood glucose measuring facility if the carbohydrate value entered by the user is greater than a carbohydrate limit.

The electronic blood glucose measuring device may further comprise at least one warning device. The carbohydrate limit may be a programmable snack size limit. The instructions stored in the memory device may include instructions that are executable by the processor to activate the at least one notification device at or near the time of controlling the display device to display instructions to measure blood glucose if the carbohydrate value entered by the user exceeds the snack size limit.

A method of setting and managing an automatic reminder in an electronic device may comprise starting a timer when an event occurs that causes an automatic reminder to be set, resetting the timer if the event occurs again before the timer times out, and activating a notification device when the timer times out. Activating a notification device may comprise activating either of an audible indication device and a vibratory device. Alternatively or additionally, activating a notification device may comprise controlling a display device to display instructions to retest the event that caused the automatic reminder to be set.

An electronic analyte measuring device may comprise a housing, a plurality of user buttons carried by the housing, a carrier port having an opening defined by the housing and extending into the housing from the opening, an analyte measuring facility positioned in the housing and in communication with the carrier port, and a processor. The analyte measuring facility may measure an analyte in a fluid sample deposited on a sample carrier that is received in the carrier port. The processor may include a memory having instructions stored therein that are executable by the processor to power up the device from a powered down state, disable the plurality of user buttons and measure the analyte in a fluid sample deposited on a sample carrier when the sample carrier is received within the carrier port with the device in the powered down state, and to enable the plurality of user buttons when the analyte measurement is complete.

The analyte measurement facility may comprise a blood glucose measurement facility that measures glucose concentration in a blood sample that is deposited on the sample carrier.

### BRIEF DESCRIPTION OF THE DRAWINGS

FIG. 1 is a block diagram of one illustrative embodiment of a wireless communication system including a medical device and a remote electronic device that are both configured to wirelessly communicate with each other.
FIG. 2 is a block diagram schematic of one illustrative embodiment of an electronic circuit that is carried by, and that controls, the remote electronic device of FIG. 1.
FIG. 3 is a block diagram schematic of some of the details of one illustrative embodiment of the memory subsystem of the remote electronic device of FIG. 2.
FIG. 4 is a diagram of one illustrative embodiment of the exterior of the remote electronic device.
FIG. 5 is a flowchart of one illustrative embodiment of a process carried out in the remote electronic device for unlocking the user buttons after detecting insertion of a sample carrier into the carrier port thereof.
FIGS. 6A - 6C depict a flowchart of one illustrative embodiment of a bolus advice process carried out by the remote electronic device.
FIG. 7 is a graphic representation of one illustrative embodiment of a bolus advice display screen produced by the process of FIGS. 6A - 6C.
FIG. 8 is a flowchart of one illustrative embodiment of a process for displaying expanded user edit areas when editing on-screen data with either of the electronic device or the medical device.
FIGS. 9A-9H are graphical representations of one illustrative embodiment of a bolus advice display screen demonstrating the use of expanding user edit areas according to the process of FIG. 7.
FIG. 10 is a flowchart of one illustrative embodiment of a process for automatically notifying and instructing a user to measure blood glucose.
FIG. 11 is a flowchart of another illustrative embodiment of a process for automatically notifying and instructing a user to measure blood glucose.
FIG. 12 is a flowchart of yet another illustrative embodiment of a process for automatically notifying and instructing a user to measure blood glucose.
FIG. 13 is a flowchart of one illustrative embodiment of a process for automatically canceling an automatic even-based notification upon reoccurrence of the event.
FIGS. 14A and 14B are diagrams of a top portion of one illustrative embodiment of a housing of the remote electronic device.

### DESCRIPTION OF THE ILLUSTRATIVE EMBODIMENTS

For the purposes of promoting an understanding of the principles of the invention, reference will now be made to a number of illustrative embodiments shown in the attached drawings and specific language will be used to describe the same.

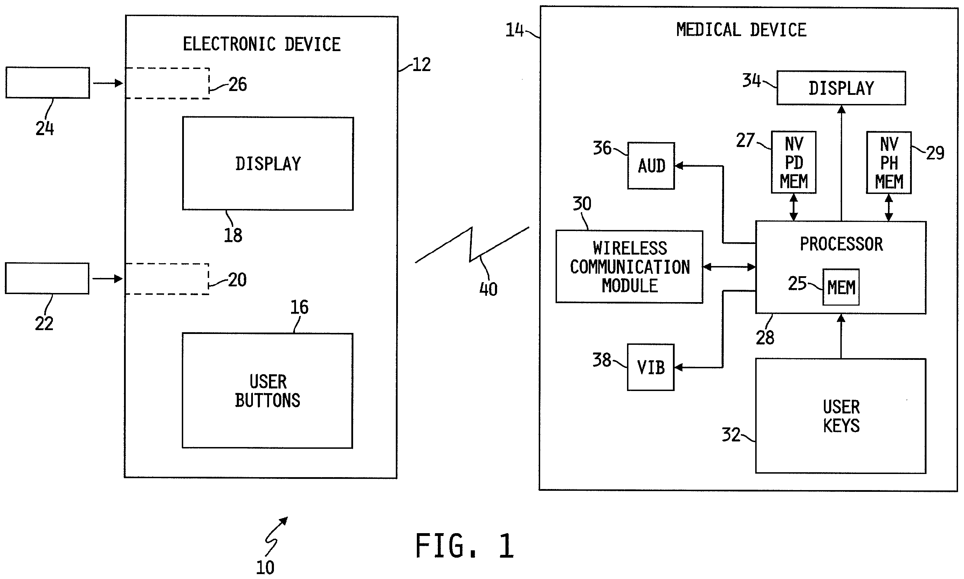

Referring now to FIG. 1, a block diagram is shown of one illustrative embodiment of a wireless communication system 10 including a remote electronic device 12 and medical device 14 that are both configured to wirelessly communicate with each other. The remote electronic device 12 has a housing through which a user button section 16 extends. In one embodiment, the user button section 16 defines a number of user buttons, keys or switches that may be manually manipulated by a user to provide input to the remote electronic device 12. A visual display unit 18 is carried by the housing of the electronic device 12, and in one embodiment the visual display unit 18 is provided in the form of a conventional liquid crystal display (LCD), although this disclosure contemplates using other conventional display units. Examples include, but are not limited to, plasma displays, light emitting diode (LED) based displays, vacuum fluorescent (VF) displays, and the like. In any case, the visual display unit 18 is controlled by the electronic device 12 to display information to a user of the device 12. In alternative embodiments, the user button section 16 may be or include one or more touch-sensitive buttons. In this embodiment, one or more touch-sensitive buttons may, but need not, form part of the display unit 18.

The electronic device 12 further includes a carrier port 20 that extends into the housing from an opening defined therein. The carrier port 20 is sized to receive therein a sample carrier or strip 22 upon which a liquid sample containing an analyte has been or will be deposited. The electronic device 12 includes electrical circuitry that analyzes the liquid sample deposited on the sample carrier 22, when the sample carrier 22 is received within the carrier port 20, to determine a concentration of the analyte contained in the liquid sample. In one embodiment, the liquid sample is blood and the analyte is glucose. In this embodiment, the sample carrier 22 may is illustratively provided in the form of a glucose test strip, and the electrical circuitry of the electronic device 12 includes conventional circuitry that measures the concentration of glucose in a blood sample deposited on the test strip 22. In alternative embodiments, the liquid sample may be or include other bodily fluids, and the analyte may be any analyte that is contained in a bodily fluid.

In the embodiment illustrated in FIG. 1, the electronic device 12 further includes a conventional data key port 26 that extends into the housing from an opening defined therein. The data key port 26 defines an electrical interface or connector therein that is configured to electrically connect to a complementarily configured electrical interface or connector defined on a conventional data key 24. The data key 24 includes a conventional memory device (not shown) that is electrically connected to the electrical interface or connector defined on the data key 24. The memory device, e.g., ROM key, is electrically connected to the electrical circuitry of the electronic device 12 via the electrical interface defined on the data key 24 and the electrical interface defined in the data key port 26 when the data key 26 is received within the data key port 24. Generally, the memory device of the data key 24 has calibration data stored therein that is specific to a lot or batch of test strips 22, and the electrical circuitry of the electronic device 12 uses the calibration data stored in the memory device of the data key 24 to correct glucose concentration measurements when using a test strip 22 from a corresponding lot or batch of test strips as is known in the art. Typically, each lot or batch of test strips 22 purchased by a user will include a dedicated data key 24 that is to be used when measuring glucose concentration with that lot or batch of strips.

It will be understood that while the carrier port 20, sample carrier 22 and electrical circuitry of the electronic device 12 have been described in one embodiment as being configured to measure the glucose concentration of blood samples deposited on the sample carrier 22, this disclosure contemplates other embodiments in which the carrier port 20, sample carrier 22 and/or electrical circuitry of the electronic device 12 is/are configured to measure other analytes in other liquid samples.

The medical device 14 includes a conventional processor 28 that is electrically connected to a wireless communication circuit 30. The processor 28 includes a conventional memory unit 25 which has stored therein a number of processes in the form of instructions that are executable by the processor 28 to control operation of the medical device 14 and to wirelessly communicate with the electronic device 12. In the illustrated embodiment, the medical device 14 further includes conventional non-volatile memory units 27 and 29. In one embodiment, the non-volatile memory unit 27 is provided in the form of a conventional ferroelectric random access memory (FRAM) and the non-volatile memory unit 29 is provided in the form of a conventional electrically erasable programmable read only memory (EEPROM), although either memory unit 27, 29 may alternatively be provided in the form of one or more other conventional non-volatile memory units. In any case, the memory units 27 and 29 are each external to the processor 28 and are each electrically connected to the processor 28. In one illustrative embodiment in which the medical device is a drug infusion pump, as will be described in greater detail hereinafter, the memory unit 27 is a pump delivery (PD) memory unit in which the processor 28 stores current pump delivery information, and the memory unit 29 is a pump history (PH) memory unit that has stored therein pump history information, e.g., in the form of event records each corresponding to an operational event of the pump 14. The medical device 14 further includes a wireless communication circuit 30 that is configured to communicate wirelessly with a similar wireless communication module of the remote electronic device 12 via a wireless communication link 40 in a conventional manner. In one embodiment, as will be illustrated by example throughout this disclosure, the wireless communication circuit 30 and the wireless communication module of the electronic device 12 are both conventional BlueTooth® modules configured to wirelessly communicate according to a conventional BlueTooth® communication protocol. It will be understood, however, that the wireless communication circuit or module 30 and the wireless communication module of the electronic device 12 may alternatively be configured to wirelessly communicate according to one or more other communication protocols.

The medical device 14 illustratively includes a housing through which a number of user keys 32 extend. The user keys 32 may be provided in the form of any number of user selectable buttons, keys or switches that are electrically connected to the processor 28. The medical device 14 further includes a visual display unit 34 that is carried by the housing and that is electrically connected to the processor 28. The visual display unit 34 may be, for example, a conventional liquid crystal display (LCD), plasma displays, light emitting diode (LED) based display, vacuum fluorescent (VF) display, or the like. The visual display unit 34 is controlled by the processor 28 to display information to a user of the medical device 14. In alternative embodiments, the user keys 32 may be or include one or more touch-sensitive buttons. In this embodiment, one or more touch-sensitive buttons may, but need not, form part of the display unit 34.

The processor 28 of the medical device 14 is further electrically connected to a conventional audible indication device 36 and to a conventional vibratory device 38. The processor 28 is generally operable to control the audible indication device 36 and the vibratory device 38 to produce one or more audible sounds and/or vibrations respectively to notify the user of various operational aspects of the medical device 14 and to also notify the user of any alarm and/or warning conditions associated with the medical device 14. In alternative embodiments, the medical device 14 may not include a display device 34 and/or user keys 32. In some such embodiments, the medical device 14 may include one or more visual indicators for conveying information to a user. Examples of such visual indicators may include, but should not be limited to, one or more lamps, one or more light emitting diodes (LEDs), or the like.

In one illustrative embodiment, the medical device 14 is an ambulatory medical device. Examples of ambulatory medical devices include, but are not limited to, an implantable liquid delivery pump or a non-implantable liquid delivery pump, such as a drug infusion pump, an implantable or non-implantable body condition sensor or sensor system, or the like. In embodiments in which the medical device 14 is a medication delivery pump, the medication delivered by such a pump may be or include, but should not be limited to, insulin or other conventional blood glucose modifying drug. In alternate embodiments, the liquid delivered by any such a pump may be or include, but should not be limited to, one or a combination of drugs, saline, one or a combination of perfusion fluids, or the like. Throughout this disclosure, the medical device 14 and operations associated with the medical device 14 will be described in the context of an insulin infusion pump, although it will be understood that the medical device 14 may alternatively be or include other medical devices and the following description therefore should not be considered to be limited to an liquid delivery pump generally or to an insulin infusion pump specifically.

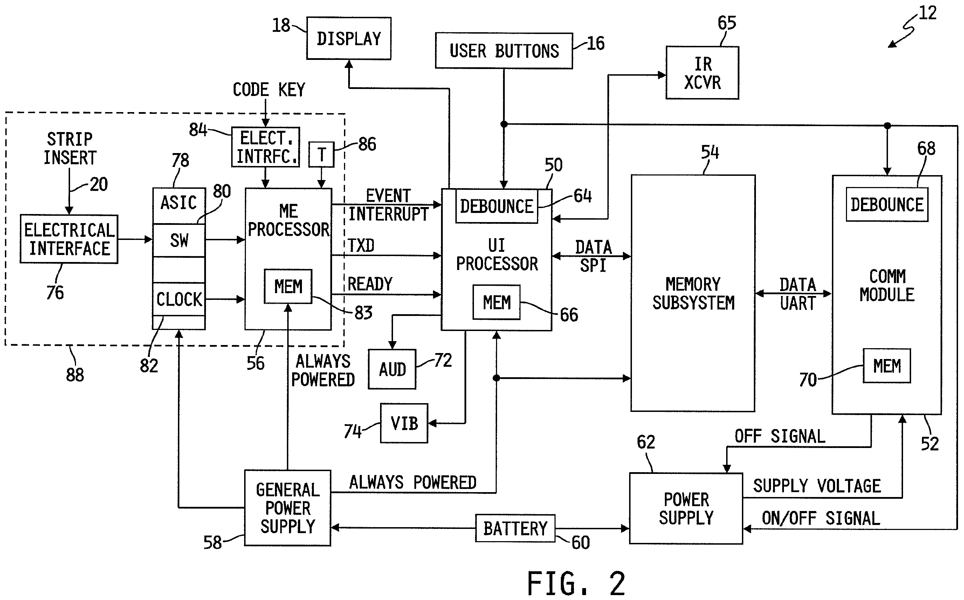

Referring now to FIG. 2, a block diagram schematic is shown of one illustrative embodiment of an electronic circuit that is carried by, and that controls, the remote electronic device 12 of FIG. 1. In the illustrated embodiment, the electronic circuit includes four modules with separate and distinct functional responsibilities. For example, the electronic circuit includes a User Interface (UI) processor 50 that is the main controller of the electronic device 12. In addition to processing all aspects of the user interfaces 16, 18, it is the origination and destination of all data communicated from and to the insulin infusion pump 14. As will be described in greater detail herein, the UI processor 50 has no control over operation of the wireless communication circuit of the remote electronic device 12. The UI processor 50 operates according to a UI clock signal that is generated internally to the UI processor 50. The UI processor 50 includes a memory unit 66 having instructions stored therein that are executable by the UI processor 50 to control operations associated with the remote electronic device 12. In one illustrative embodiment, the UI processor 50 is a UPD70F3719GC 32-bit microcontroller that is commercially available from NEC Electronics America of Santa Clara, California, although this disclosure contemplates other implementations of the UI processor 50.

The electronic circuit of FIG. 2 further includes a wireless communication circuit 52 that is exclusively responsible for the control of all wireless communications with one or more external electronic devices but that does not control any other operations associated with the electronic device 12. The wireless communication circuit 52 operates from a clock signal that is generated internally to the wireless communication circuit 52 and that is not synchronized to the UI clock signal from which the UI processor 60 operates. Operation of the wireless communication circuit 52 is therefore asynchronous with respect to the operation of the UI processor 60. In one illustrative embodiment, the wireless communication circuit 52 is provided in the form of a conventional BlueTooth® telemetry module that includes a conventional processor and a memory unit 70, and that further includes conventional wireless communication hardware such as a suitable antenna. The memory unit 70 illustratively has stored therein instructions that are executable by the processor of the wireless communication circuit 52 to exclusively control of all wireless communications with external devices, such as the insulin infusion pump 14. In one illustrative embodiment, the wireless communication circuit 52 is a BC419143B BlueCore™4-Flash Plug-n-Go™ single chip BlueTooth® radio and baseband integrated circuit for BlueTooth® 2.4 GHz systems that is commercially available from CSR of Richardson, Texas, although this disclosure contemplates other implementations of the wireless communication circuit 52. Alternatively, as described hereinabove, this disclosure contemplates embodiments in which the wireless communication module 52 is configured for wirelessly communication according to wireless communication protocols other than BlueTooth®.

As illustrated in FIG. 2, the UI processor 50 and the wireless communication module 52 each include debounce circuitry 64 and 68 respectively that is electrically connected to the user buttons 16. The debounce circuitry 64, 68 is conventional in that it reduces the sensitivity of the processors 50 and 52 to spurious switching events associated with the user buttons 16, thereby increasing the likelihood that only actual button presses are detected by the processors 50 and 52.

The electronic circuit illustrated in FIG. 2 further includes a memory subsystem 54 that is electrically connected to the UI processor 50 and also to the wireless communication circuit 52. The memory subsystem 54 is generally operable to store, at least temporarily, data moving between the UI processor 50 and the wireless communication circuit 52. Data communication between the memory subsystem 54 and the UI processor 50 is illustratively carried out via a serial peripheral interface, SPI, in which case the transfer of data between the memory subsystem 54 and the UI processor 50 is synchronous with a data transfer clock, SCLK, of the UI processor 50. Illustratively, data communication between the memory subsystem 54 and the wireless communication circuit 52 is carried out via a universal asynchronous receiver/transmitter (UART) interface, in which case the transfer of data between the memory subsystem 54 and the wireless communication circuit 52 is asynchronous. In some alternative embodiments, the data transfer interfaces may be interchanged such that data transfer between the memory subsystem 54 and the UI processor 50 is asynchronous and data transfer between the memory subsystem 54 and the wireless communication circuit 52 is synchronous.

The memory subsystem 54 temporarily stores data moving between the UI processor 60 and the wireless communication circuit 52. In some embodiments, the memory subsystem 54 does not control other circuitry, and in some such embodiments the memory subsystem 54 may be provided in the form of a conventional memory device. In other embodiments in which the memory subsystem 54 does or does not control other circuitry, the memory subsystem 54 may be provided in the form of a conventional processor that is configured to operate as a Dual-Port RAM (DPR) processor. In such embodiments, the DPR processor 54 operates from a clock signal that is separate from the UI clock signal from which the UI processor 60 operates. In one illustrative embodiment, such a DPR processor 54 is a MC9S08GT16A 8-bit microcontroller unit that is commercially available from Freescale Semiconductor, Inc. of Austin, Texas, although this disclosure contemplates other implementations of the memory subsystem 54 that is provided in the form of a conventional processor configured as a DPR processor 54.

The electronic circuit illustrated in FIG. 2 further includes a Measurement Engine (ME) processor 56 that controls analyte concentration measurements of liquid samples contained on test elements 22, e.g., blood glucose measurements, and that reports the analyte concentration measurement results to the UI processor 50. The ME processor 56 includes a memory unit 83 having instructions stored therein that are executable by the ME processor 56 to control analyte measurement operations. The ME processor 56 operates from an internally generated clock signal that is separate from the clock signal from which the UI processor 50 operates. The ME processor 56 is electrically connected to the UI processor 50 via an Event Interrupt line, TXD (data transmission) line and a Ready line. The Event Interrupt line is illustratively used by the ME processor 56 to notify the UI processor of analyte measurement events, such as a strip insert event in which a user initiates an analyte measurement. The TXD line is used by the ME processor 56 to transmit analyte measurement data to the UI processor 50 for display on the display unit 18, for storage thereof in a history database and/or for use in conducting other operations. The Ready line is used by the ME processor 56 to notify the UI processor 50 of the operational state, e.g., measuring or not measuring analyte concentration, of the ME processor. In one illustrative embodiment, the ME processor 56 is a MSP430T2AIPEG mixed-signal microcontroller unit that is commercially available from Texas Instruments, Inc. of Dallas, Texas, although this disclosure contemplates other implementations of the ME processor 56.

As illustrated in FIG. 2, the ME processor 56, along with other electrical components, form an analyte measuring facility 88, e.g., a glucose meter. In addition to the ME processor 56, the analyte measuring facility 88 further includes an application specific integrated circuit (ASIC) 78 that is electrically connected to the ME processor 56 and also to an electrical interface 76 within the carrier port 20. In one illustrative embodiment, when a sample carrier 22, e.g., a glucose test strip, is inserted into the carrier port 20, electrical contacts on the sample carrier 22 contact the electrical interface 76 to thereby electrically connect the sample carrier 22 to the ASIC 78. A switch 80 contained in the ASIC is triggered by insertion of the carrier 22 into the carrier port 20, and an output of the switch 80 thus notifies the ME processor 56 of insertion of a carrier 22 into the carrier port 20. The ASIC 78 further illustratively includes a clock circuit 82 that is programmable for a number of different functions. For example, the clock circuit 82 may be programmed to generate a signal to automatically turn on, e.g., power up, the device 12 at one or more programmable times. As another example, the clock circuit 82 may be programmed to generate a signal corresponding to one or more reminders. Other examples will occur to those skilled in the art, and such other examples are contemplated by this disclosure. In any case, the signal generated by the clock circuit 82 is provided to the ME processor 56, and the ME processor 56 is responsive to the receipt of this signal to power up from a sleep state if the ME processor 56 is in such a sleep state, and to produce an event interrupt signal on the Event Interrupt line. The event interrupt signal is received by the UI processor 50, which then powers up from a sleep state if the UI processor 50 is in such a sleep state, and/or generates an audible or visible reminder corresponding to any reminder time programmed in the clock circuit 82.

As illustrated in FIG. 2, the analyte measuring facility 88 further includes another electrical interface 84 that is positioned within the code key port 26. Illustratively, when a code key 24 is received within the code key port 26, electrical contacts on the code key 24 electrically connect with the electrical interface 84 so that the ME processor 56 may read the calibration information stored in the memory device of the code key 24. The analyte measuring facility 88 further includes a temperature sensor 86 that is electrically connected to the ME processor 56. In one illustrative embodiment, the temperature sensor 86 is provided in the form of a conventional thermistor, although this disclosure contemplates other embodiments in which the temperature sensor 88 may be or include one or more other conventional temperature sensors. In any case, the ME processor 56 is operable to receive a temperature signal from the temperature sensor 86 that corresponds to an operating temperature of the analyte measuring facility. In one illustrative embodiment, the memory 83 has instructions stored therein that are executable by the ME processor 56 to disable, i.e., not conduct, analysis of an analyte containing sample if the temperature signal produced by the temperature sensor 86 indicates that the temperature of the analyte measuring facility 88 is less than a threshold temperature. In such cases, the ME processor 56 is further operable, pursuant to the instructions stored in the memory 83, to inform the UI processor 50 that the analyte measuring facility is so disabled, and the UI processor 50 is operable, pursuant to instructions stored in the memory unit 66, to control the display device 18 to display a message indicating that the temperature is too low to conduct analyte concentration measurements.

The electronic circuit illustrated in FIG. 2 further includes a general power supply 58 that provides a supply voltage to the ASIC 78, the ME processor 56, the UI processor 50 and the memory subsystem 54 on a continuous basis. The supply voltage is derived by the general power supply circuit 58 from one or a series or parallel combination of rechargeable or non-rechargeable batteries (BATTERY) 60.

A dedicated power supply 62 provides a supply voltage, which is also derived from the one or series or parallel combination of rechargeable or non-rechargeable batteries (BATTERY) 60, to the wireless communication module 52. The power supply 62 receives one control input from the user buttons 16, and in the illustrated embodiment the power supply 62 may be powered on and off via one or a combination of the user buttons 16 via the one control input. The power supply 62 also receives another control input from the wireless communication circuit 52, and in the illustrated embodiment the power supply 62 may be turned off by the wireless communication circuit 52 via the other control input.

In addition to the display 18, the UI processor 50 is electrically connected to a conventional audible indication device 72 and also to a conventional vibratory device 74. The UI processor 50 is generally operable to control the audible indication device 72 and the vibratory device 74 to produce one or more audible sounds and/or vibrations respectively to provide for the capability of the device 12 to produce corresponding audible and/or tactile notifications, i.e., alarms or the like. In one embodiment, the audible indication device 72 is a tone generator that produces a beep or other tone when activated, although the audible indication device 72 may alternatively or additionally be or include one or more other conventional audible indication devices.

In the illustrated embodiment, the UI processor 50 is also electrically connected to a conventional infrared (IR) transceiver 65 that is configured to operate at an infrared wavelength. The UI processor 50 is configured to control the IR transceiver 65 is a conventional manner to transmit wireless signals to an off-board electronic device such as a personal computer (PC), laptop or notebook computer, personal data assistant (PDA) or other computer-based system. The off-board electronic device includes an IR transceiver and other circuitry and/or software via which the off-board electronic device can wirelessly communicate with the UI processor 50 through the IR transceiver 65. Wireless signals transmitted by such an IR transceiver of an off-board electronic device are received by the UI processor 50 via the IR transceiver 65. In the illustrated embodiment, the UI processor 50 is operable to communicate with an off-board electronic device via the IR transceiver 65 to upload information, e.g., measured analyte information and/or delivered drug information, to the off-board electronic device for subsequent analysis and/or to download control software or the like from the off-board electronic device. In alternative embodiments, the transceiver 65 may be configured to operate at a visible or ultraviolet wavelength.

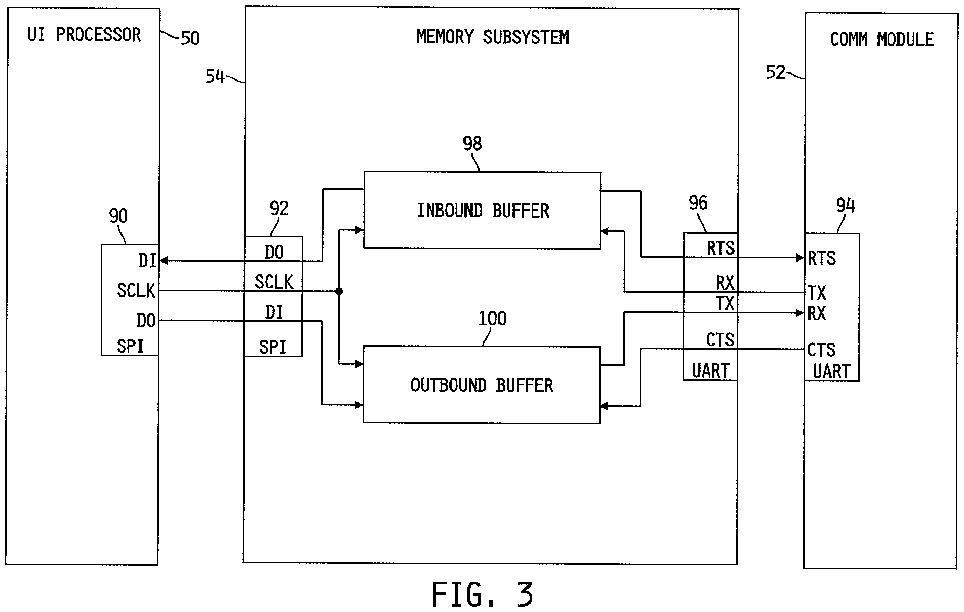

Generally, the memory subsystem 54 acts as an independent repository of data packets moving between the UI processor 50 and the wireless communication circuit 52. Referring to FIG. 3, a block diagram of some of the details of the memory subsystem 54 is shown along with electrical connections to the UI processor 50 and to the wireless communication circuit 52. In the illustrated embodiment, the memory subsystem 54 is provided in the form of a DPR processor as described above, and FIG. 3 will be described in this context, although it will be understood that the memory subsystem 54 may alternatively be provided in other forms as described above.

In the embodiment illustrated in FIG. 3, one of the dual ports of the DPR processor 54 is a serial peripheral interface (SPI) port 92 that is electrically connected to a serial peripheral interface port 90 of the UI processor 50 via a conventional serial communications interface. The serial communications interface operates from a serial clock signal, SCLK, (e.g., 125 kHz) that is derived from the UI clock signal. Transfer of inbound and outbound data between the SPI port 90 of the UI processor 50 and the SPI port 92 of the DPR processor 54 is controlled by the UI processor 50 using the serial clock signal, SCLK, so that data transfer between the two processors 50, 54 is synchronized.

The other of the dual ports of the DPR processor 54 is a universal asynchronous receiver/transmitter (UART) port 96 that is electrically connected to a UART port 94 of the wireless communication circuit 52 via a conventional asynchronous interface. Transfer of inbound and outbound data packets between the UART port 94 of the wireless communication circuit 52 and the UART port 96 of the DPR processor 54 (e.g., at 150 kbps) is controlled by the wireless communication circuit 52, and takes place asynchronously with respect to the transfer of inbound and outbound data between the SPI port of the UI processor 50 and the DRP processor 54.

The DPR processor 54 has an inbound data buffer 98 and an outbound data buffer 100 that are each accessible by the SPI and UART ports 92 and 96 respectively of the DPR processor 54. The UART port 96 of the DPR processor 54 includes conventional clear to send (CTS) and ready to send (RTS) lines. The CTS line is monitored by the DPR processor 54 and the RTS line is monitored by the wireless communication circuit 52. The DPR processor 54 deactivates the UART RTS line whenever the inbound data buffer 100 is full, and otherwise activates the UART RTS line. The wireless communication circuit 52 activates the UART CTS line whenever the UART port of the wireless communication circuit 52 is requesting data, and otherwise deactivates the UART CTS line.

When data is to be sent by the UI processor 50 to an external device or system, e.g., the insulin infusion pump 14, the UI processor 50 first requests the state of the outbound data buffer 100 of the DPR processor 54. If the DPR processor 54 answers that its outbound data buffer 100 is "not full," the UI processor 50 transfers the data, or as much of the data as possible, to the outbound data buffer 100 of the DPR processor 54 via the data out (DO) line of the SPI port 90 at a rate determined by SCLK. If the DPR processor 54 instead answers that the outbound data buffer 100 is "full," the UI processor 50 waits for a time interval and then repeats the process of requesting the state of the outbound data buffer 100, etc.

Periodically with respect to the clock signal of the wireless communication circuit 52 and asynchronously with respect to the SCLK signal, the wireless communication circuit 52 requests data from the DPR processor 54 by activating the UART CTS line of the DPR processor 54. As long as the outbound data buffer 100 of the DPR processor 54 is empty, the wireless communication circuit 52 continues to periodically activate the UART CTS line. If the UART CTS line is active and the outbound data buffer 100 of the DPR processor 54 is not empty, the wireless communication circuit 52 retrieves the data from the outbound data buffer 100 of the DPR processor 54 via the RX line of the UART port 96. The DPR processor 54 illustratively transfers the data stored in its outbound data buffer 100 to its UART port 96 in a first received to last received order until the outbound data buffer 100 has been emptied or until the wireless communication circuit 52 deactivates the UART CTS line. The wireless communication circuit 52 then incorporates the data retrieved from the outbound data buffer 100 of the DPR processor 52, via the data UART, into to the wireless communication protocol structure, and wirelessly transmits the incorporated data via conventional wireless signal transmission circuitry contained within the wireless communication module 52. The wireless communication circuit 52 does not process, interpret or alter the contents of the data retrieved from the outbound data buffer 100 of the DPR processor 54, nor does it make any decisions or execute any steps based on the contents of the data. Rather, the wireless communication circuit 52 treats all such data the same, regardless of its contents, by incorporating the data into a predefined wireless communication protocol structure, e.g., BlueTooth® protocol structure, and then wirelessly transmitting the incorporated data using the predefined wireless communication protocol. Information transferred by the UI processor 50 to the memory subsystem 54, and then from the memory subsystem 54 to the wireless communication circuit 52 for wireless transmission to another electronic device is thus referred to as outbound information or data.

Inbound wireless signal transmissions from external devices or systems, e.g., the insulin infusion pump 14, are received by the wireless communication circuit 52 via conventional wireless signal receiving circuitry of the wireless communication circuit 52. The wireless communication circuit 52 first isolates the inbound data from the wireless communication protocol structure, and then checks the status of the UART RTS line of the DPR processor 54. If the RTS line is activated, indicating that the inbound data buffer 98 of the DPR processor 54 is not full, the wireless communication circuit 52 sends the isolated data, or as much if the data as possible, to the UART port 96 of the DPR processor 54. The DPR processor 54 then places the data received at the UART port 96 into the inbound data buffer 98 of the DPR processor 54. If the UART RTS line is deactivated, indicating that the inbound data buffer 98 of the DPR processor 54 is full, the wireless communication circuit 52 waits for a time interval before rechecking the state of the UART RTS line.

Periodically, and asynchronously with respect to the operation of the wireless communication circuit 52, the UI processor 50 requests the state of the inbound data buffer 98 of the DPR processor 54 via the data in (DI) line of the SPI port 90. As long as the DPR processor 54 answers that the inbound data buffer 98 is empty, the UI processor 50 continues to periodically request the state of the inbound data buffer 98. If the DPR processor 54 answers that the inbound data buffer 98 of the DPR processor 54 contains data, the UI processor 50 retrieves the data from the inbound data buffer 98 of the DPR processor 52 via the data in (DI) line of the SPI port 90 using the SCLK signal, and then processes the data according to its contents. "Checking" the inbound and/or outbound data buffer 98, 100 of the DPR processor 54 by the wireless communication circuit 52 and/or UI processor 50, as this term may be used hereinafter, will generally refer to the process just described in the preceding several paragraphs. While FIGS.2 and 3 illustrate an embodiment in which the interface between the UI processor 50 and the memory subsystem 54 is a synchronous interface and the interface between the wireless communication circuit 52 and the memory subsystem 54 is an asynchronous interface, this disclosure contemplates alternative embodiments in which the interface between the UI processor 50 and the memory subsystem 54 is an asynchronous interface and the interface between the wireless communication circuit 52 and the memory subsystem 54 is an synchronous interface or in which both interfaces are asynchronous or synchronous interfaces. In any case, it should be apparent that the UI processor 50 at all times operates independently and asynchronously with respect to the operation of the wireless communication circuit 52, and the wireless communication circuit 52 operates independently and asynchronously with respect to the operation of the UI processor 50 and also with respect to the operation of the DPR processor 54.

The UI processor 50 controls the display 18 of the electronic device 12 to indicate the connection status of the wireless communication module 52 relative to the wireless telemetry system of the insulin infusion pump 14. Upon power up of the electronic device 12, following activation of the power supply 62 via the user buttons 16 after being deactivated and under certain other operating conditions that will be described in greater detail hereinafter, the UI processor 50 attempts to establish a wireless connection with the insulin infusion pump 14. While a wireless connection is not established between the electronic device 12 and the insulin infusion pump 14, the UI processor 50 controls the display 18 to display a flashing (or fixed) icon to indicate that no wireless connection exists between the electronic device 12 and the insulin infusion pump 14. The UI processor 50 independently controls the display 18 in this manner without any information provided by the wireless communication module 52. The UI processor 50 then initiates establishment of a wireless connection between the remote electronic device 12 and the insulin infusion pump 14 by placing a message into the data buffer 100 of the outbound port of the memory subsystem 54, as described above. In this case, the message includes a wireless connection request, e.g., in the form of a command to transmit an acknowledgement response back to the electronic device 12. The wireless communication circuit 52 then transmits this message as described above. If the insulin infusion pump 14 is within range, the insulin infusion pump 14 receives the message and responds to the wireless connection request by wirelessly transmitting a message that includes an acknowledgement response. If the transmitted message is received by the electronic device 12, the wireless communication circuit 52 is operable as described above to isolate the message from the wireless communication protocol structure and to place the message in the data buffer 98 of the inbound port of the memory subsystem 54. The UI processor 50 then retrieves the message from the inbound port of the memory subsystem 54, processes the message to determine whether it contains an acknowledgement response. If the message contains an acknowledgement response, the UI processor 50 interprets this as indicating that a wireless connection is now established between the electronic device 12 and the insulin infusion pump 14, and controls the display device 18 to display a fixed (or flashing) icon to indicate that a wireless connection is established between the electronic device 12 and the insulin infusion pump 14. The electronic device 12 periodically transmits a wireless connection status message to the infusion pump 14 in the above fashion at regular intervals. As long as the insulin infusion pump 14 responds as just described, the UI processor 50 controls the display 18 to display the fixed (or flashing) icon to indicate that a wireless connection exists between the electronic device 12 and the insulin infusion pump 14. If the UI processor 50 does not receive such a response within a predefined time period following storage of the acknowledgement response in the memory subsystem 52, the UI processor 50 controls the display 18 to display a flashing (or fixed) icon indicating that the wireless connection between the electronic device 12 and the insulin infusion pump 14 does not exist or no longer exists.

In the illustrated embodiment the power supply 62 is generally powered on as long as the wireless communication circuit 52 is communicating with either or both of the UI processor 50 or the insulin infusion pump 14, unless otherwise powered off manually by a user via the user buttons 16 or automatically by the wireless communication circuit 52. For example, the power supply 62 may be completely powered down, i.e., turned off, from any state via a simultaneous or sequential user press of a number of the user buttons 16. The power supply 62 remains in the completely powered down state until the user again presses the simultaneous or sequential number of the user buttons 16 or a different simultaneous or sequential user press of a number of the user buttons, or if the user powers down the electronic device 12 and then powers back up the electronic device 12.

While the power supply 62 is on and supplying the supply voltage to the wireless communication circuit 52, the wireless communication circuit 52 is responsive to a number of different events to transition itself into, and out of, any of a plurality of different low power states, and to also turn off the power supply 62 after being in a lowest power sleep state for a predefined time period of inactivity. For example, when in a fully powered "awake" state, the wireless communication circuit 52 is operable to periodically, e.g., every 100-200 milliseconds, check the outbound data buffer 100 of the memory subsystem 54 as described above. As another example, each time the wireless communication circuit 52 finds data to be sent in the outbound data buffer 100 of the memory subsystem 54, the wireless communication circuit 52 incorporates the data into the predetermined wireless communication protocol structure, and wirelessly transmits corresponding signals to the insulin infusion pump 14 as described above. The wireless communication circuit 52 transitions to a first low power state if it fails to find data in the outbound data buffer 100 of the memory subsystem 54 when a predefined time period elapses since last finding data in the outbound data buffer 100. Thereafter, the wireless communication circuit 52 transitions to successively lower power states as successively longer time periods elapse since last finding data in the outbound data buffer 100. The number of different power states generally range between full (100%) power and a lowest power "deep sleep" state, and may include any number of reduced power states between these two extremes. When in the lowest power "deep sleep" state, the wireless communication circuit 52 periodically, e.g., every 400 milliseconds, wakes up to a "UART only" state, in which the wireless communication circuit 52 has sufficient power to check the status of the outbound data buffer 100 of the memory subsystem 54 via the data UART line. If the outbound data buffer 100 of the memory subsystem 54 has data stored therein, the wireless communication circuit 52 wakes up to a full power state to service the data. If the outbound data buffer 100 of the memory subsystem 54 has no data stored therein, the wireless communication circuit 52 transitions back to the lowest power "deep sleep" state. After being in the lowest power sleep state for a predefined period of time of inactivity, the wireless communication circuit 52 sends a control signal to the power supply 62 that causes the power supply 62 to turn off. As a further example, the wireless communication circuit 52 directly monitors activity of the user buttons 16 via the debounce circuitry 68, and when the wireless communication circuit 52 detects user press of the ON button, the wireless communication processor transitions itself from any of the lower power states to the full power state. Thus, in the lowest power "deep sleep" state, the wireless communication circuit 52 must be capable of monitoring at least the ON button of the user buttons 16. Similarly, when the wireless communication circuit 52 detects user press of the OFF button, the wireless communication circuit 52 transitions itself from any of the power states to the lowest power "deep sleep" state.

When a wireless connection is established between the electronic device 12 and the insulin infusion pump14, and the UI processor 50 determines that the wireless connection should be terminated, the UI processor 50 stores a message in the outbound data buffer 100 of the memory subsystem 54 that contains a connection termination request. When the wireless communication circuit 52 thereafter finds the message in the outbound data buffer 100 of the memory subsystem 54, the wireless communication circuit 52 incorporates the message into the predetermined wireless communication protocol and then transmits the message via its wireless communication circuitry to the insulin infusion pump14. The insulin infusion pump 14 then wirelessly sends a signal containing a predefined connection termination response back to the remote electronic device 12. Subsequently the processor 28 instructs the wireless communication circuit 30 to orderly terminate communications or connections with the wireless communications circuit 52' that may be specific to the predetermined wireless communications protocol. When the wireless connection is terminated in this manner, the wireless communication circuit 52 is operable to periodically, but asynchronously with respect to operation of the UI processor 50, check the outbound data buffer 100 of the memory subsystem 54. If no data resides in the outbound data buffer 100, the wireless communication circuit 52 successively enters lower power sleep states or modes as described above. If, however, the wireless communication circuit 52 finds data in the outbound data buffer 100 of the memory subsystem 54, the wireless communication circuit 52 attempts to establish (or re-establish) a wireless connection with the wireless communication circuit 30 of the insulin infusion pump 14 as described above.

If, after a predefined or programmed number of attempts and/or elapsed time, no wireless connection can be established between the wireless communication circuit 52 and the wireless communication circuit 30, the wireless communication circuit 52 illustratively clears the outbound data buffer 100 of the memory subsystem 54. Alternatively, the UI processor 50 may clear the outbound data buffer 100 if it determines that data exists in the outbound data buffer 100 after some time period has elapsed since storing the wireless communication message in the outbound data buffer 100 or after some time period has elapsed after determining, based on failure to receive acknowledgements from the insulin infusion pump 14, that a wireless connection between the remote electronic device 12 and the insulin infusion pump 14 no longer exists. In any case, with the outbound data buffer 100 of the memory subsystem 54 empty, the wireless communication circuit 52 successively enters lower power sleep states or modes as described above.

In the event of a lost wireless connection between the remote electronic device 12 and the insulin infusion pump 14, the wireless communication circuit 52 is operable in one embodiment to turn off its wireless transmission circuitry and to transition to a low power state if it fails to find data in the outbound data buffer 100 of the memory subsystem 54 since last finding data in the outbound data buffer 100. Because the wireless connection is lost, the UI processor 50 will no longer receive acknowledgements from the insulin infusion pump 14 and will therefore cease to store messages in the outbound data buffer 100 of the memory subsystem 54. However, a message, or at least part of a message, may reside within the outbound data buffer 100 when the wireless connection is lost. In this case, after a predefined or programmed number of attempts and/or after a predefined or programmed elapsed time, no wireless connection can be established with the insulin infusion pump 14, the wireless communication circuit 52 illustratively clears the outbound data buffer 100 of the memory subsystem 54. Alternatively, the UI processor 50 may clear the outbound data buffer 100 if it determines that data exists in the outbound data buffer 100 after some time period has elapsed since last storing a message in the outbound data buffer 100 or after some time period has elapsed after determining, based on failure to receive acknowledgements from the insulin infusion pump 14, that a wireless connection between the devices 12 and 14 no longer exists. In any case, with the outbound data buffer 100 of the memory subsystem 54 empty, the wireless communication circuit 52 successively enters lower power sleep states or modes as described above.

In one illustrative embodiment, the UI processor 50 and the processor 28 of the insulin infusion pump 14 may use scheduled messages and internal timers to control determinations by each of whether a wireless connection between the remote electronic device 50 and the insulin infusion pump 14 exists. For example, during information exchange between the electronic device 12 and the insulin infusion pump 14, the UI processor 50 is operable to periodically, e.g., every 100 milliseconds, transfer a message to the outbound data buffer 100 of the memory subsystem 54 and to reset an internal timer circuit. The wireless communication circuit 52 asynchronously retrieves the message from the outbound data buffer 100 of the memory subsystem 54 and transmits the message to the insulin infusion pump 14 as described above. The insulin infusion pump 14 is responsive to receipt of the message to immediately transmit a message back to the electronic device 12 that contains an acknowledgement. The message transmitted by the insulin infusion pump 14 is received and unpacked from the wireless communication protocol by the wireless communication circuit 52, and then stored by the wireless communication circuit 52 in the inbound data buffer 98 of the memory subsystem 54. The UI processor 50 then retrieves the message from the inbound data buffer 98 of the memory subsystem 54 and processes the message to determine whether it contains an acknowledgement. As long as an acknowledgement is received by the UI processor 50 in this manner before the next scheduled transfer of a message to the outbound data buffer 100 of the memory subsystem 54, the UI processor 50 resets its internal timer circuit when transferring the next message to the memory subsystem 54. However, if an acknowledgement is not received by the UI processor 50 before the next scheduled transfer of a message to the outbound data buffer 100 of the memory subsystem 54, the UI processor 50 transfers the message to the outbound data buffer 100 of the memory subsystem 54 without resetting its internal timer circuit. If no acknowledgement is received by the UI processor 50 within a predefined or programmed time period, e.g., 1-2 minutes, the internal timer circuit of the UI processor 50 times out and the UI processor 50 stops transferring messages to the outbound data buffer 100 of the memory subsystem 54. The insulin infusion pump 14, in this embodiment, ceases to send acknowledgements back to the remote electronic device 12 after a predefined or programmed time period, e.g., 2 minutes, has passed without receiving a message transmitted by the electronic device 12.

Illustratively, the UI processor 50 is operable to cease storing messages in the outbound data buffer 100 of the memory subsystem 54 upon detection of insertion of a sample carrier 22 into the carrier port 20 as described above. After a predefined time period in which the wireless communication circuit 52 thereafter fails to find such messages in the outbound data buffer 100 of the memory subsystem 54, the wireless communication circuit 52 begins transitioning to lower power states as described above. When the UI processor 50 then resumes storing messages in the outbound data buffer 100 of the memory subsystem 54 after the analyte measurement is complete, the wireless communication circuit 52 wakes up to full power to service it. This may take at least a wake up time period, e.g., as much as 400 milliseconds, if the wireless communication circuit 52 has just entered the lowest power "deep sleep" state when the first message is stored in the outbound data buffer 100 of the memory subsystem 54 after the analyte measurement is complete.

Unless the remote electronic devices 12 and the insulin infusion pump 14 are communicating information, the wireless communication circuit 52 is generally in one of the lower power sleep states or modes. When insertion of a sample carrier 22 into the carrier port 20 is detected, the electronic device 12 performs an analyte determination test as described above. The electronic device 12 generally does not wirelessly communicate with the insulin infusion pump 14 during the analyte determination test, and the wireless communication circuit 52 is thus typically in one of the lower power sleep states when insertion of the sample carrier 22 into the carrier port 20 is detected. Because the UI processor 50 stops storing messages in the outbound data buffer 100 of the memory subsystem 54 when insertion of the sample carrier 22 into the carrier port 20 is detected, the wireless communication circuit 52 therefore typically enters successively lower power sleep states after insertion of the sample carrier 22 into the carrier port 20 is detected.

While the electronic device 12 is illustrated and described above with respect to FIGS. 1-3 as including an analyte measuring facility 88, such an analyte measuring facility may be omitted in alternative embodiments. In any case, the electronic device 12 and the insulin infusion pump 14 may illustratively be paired according to a pairing process that establishes secure communications between the electronic device 12 and the insulin infusion pump 14. Illustratively, this process may be carried out to initially establish secure wireless communications between the electronic device 12 and a particular insulin infusion pump 14, and then again if the electronic device 12 is to be paired with a different insulin infusion pump 14 or vice versa. In one illustrative embodiment, the electronic device 12 may only be paired with a single insulin infusion pump 14 at a time, although this disclosure contemplates other embodiments in which the electronic device 12 may be paired with any number of medical devices 14 generally and/or other electronic devices, and/or in which the medical device 14 may be paired with any number of electronic devices 12 or other medical devices.

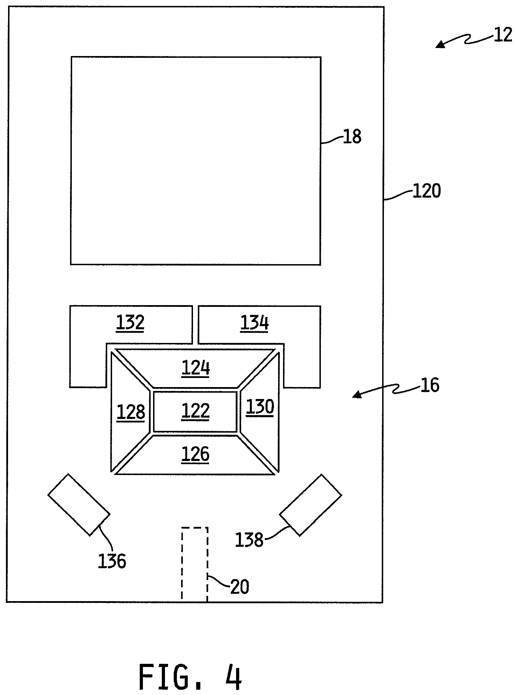

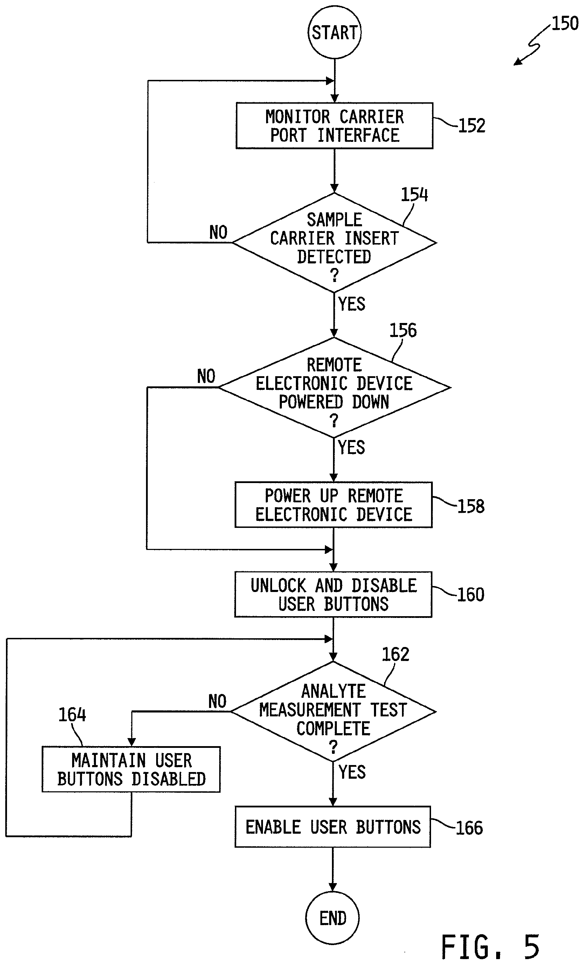

Referring now to FIG. 4, a diagram is shown of one illustrative embodiment of the exterior of the remote electronic 12. In the illustrated embodiment, the remote electronic device 12 includes a housing 120 to which the display device 18 and the user buttons 26 are mounted. In the embodiment illustrated in FIG. 5, the user buttons 26 include an ENTER key 122, an up key 124, a down key 126, a left key 128 and a right key 130, wherein the keys 124, 126, 128 and 130 are configured to provide for up, down, left and right navigation respectively through application screens displayed on the display device 18. The user buttons 26 further include two so-called "soft" keys 132 and 134 that may be programmed to provide desired functions, as well as an on/off button 136 and a display backlight activation button 138. In the illustrated embodiment, the carrier port 20 is positioned substantially centrally at one end of the device 12 such that the opening of the carrier port 20 is positioned in-line with the up and down buttons 120 and 124, although this disclosure contemplates embodiments in which the carrier port 20 is alternatively positioned on the device 12.

Referring now to FIG. 5, a flowchart is shown of one illustrative embodiment of a process 150 that is carried out in the remote electronic device 12 for unlocking the user buttons 26 after detecting insertion of a sample carrier 22 into the carrier port 20. The process is illustratively stored in the memory unit 66 of the remote electronic device 12 in the form of instructions that are executable by the UI processor 50 to carry out the process 150. The process 150 begins at step 152 wherein the UI processor 50 is operable to monitor the electrical interface 76 of the carrier port 20. Thereafter at step 154, the UI processor 50 is operable to determine whether a sample carrier 22 has been inserted into the carrier port 20. If not, step 154 loops back to the beginning of step 152.

In one embodiment, the UI processor 50 is operable at steps 152 and 154 to monitor the electrical interface 76 via the ME processor 56 and the ASIC 78. As described hereinabove, the switch 80 of the ASIC is operable to provide a strip insert signal to the ME processor 56 upon detection of engagement of an electrical interface of a sample carrier 22 with the electrical interface 76 when the sample carrier 22 is inserted into the carrier port 20 of the remote electronic device 12. The ME processor 56 is, in turn, responsive to the strip insert signal produced by the switch 80 of the ASIC 78 to notify the UI processor 50 of the event via, for example, the Event Interrupt line. In some alternative embodiments, the UI processor 50 may be configured to directly monitor the electrical interface 76, and in other alternative embodiments the UI processor 50 may be configured to determine whether a sample carrier 22 has been inserted into the carrier port 20 by monitoring one or more conventional position or proximity sensors or the like.

From the "YES" branch of step 154, the process 150 advances to step 156 where the UI processor 50 is operable to determine whether the remote electronic device 12 is currently powered down. Illustratively, the UI processor 50 is operable to execute step 156 by monitoring an internal operating state indicator, although the UI processor 50 may alternatively be operable at step 156 to determine the operating state of the remote electronic device according to other conventional techniques. In any case, if the UI processor 50 determines at step 156 that the remote electronic device 12 is powered down, the UI processor 50 is operable to power up the remote electronic device in a conventional manner. From the "NO" branch of step 156 and from step 158, the process 50 advances to step 160 where the UI processor 50 is operable to unlock and disable the user buttons 26. In one embodiment, the UI processor 50 is operable at step 160 to unlock the user buttons 26 regardless of whether they were manually locked previously by a user. Alternatively, the UI processor 50 may be operable at step 160 to unlock the user buttons 26 only if they have been previously manually locked by a user. In either case, the UI processor 50 is operable at step 160 to disable the user buttons 26 such that subsequent pressing of any of the user buttons 26 will not be acknowledged or acted upon by the UI processor 60.

The process 150 advances from step 160 to step 162 where the UI processor 50 is operable to determine whether the analyte measurement test is complete. Illustratively, the UI processor 50 is operable at step 162 to determine that the analyte measurement test is complete when the ME processor 56 provides a measured glucose value to the UI processor 50 for display on the display unit 18, and/or when the ME processor 56 otherwise signals to the UI processor 50 via the Ready line or Event Interrupt line that the analyte measurement test is complete. For purposes of the process 150, the UI processor 50 may further be operable at step 162 to determine that the analyte measurement test is complete if the user removes the sample carrier 22 from the carrier port 20 before the analyte measurement test is complete. In such cases, the UI processor 50 is operable to determine whether the sample carrier 22 has been removed from the carrier port 20 before the analyte measurement test is complete using any of the techniques just described for determining whether a sample carrier 22 has been inserted into the carrier port 20. In any case, if the UI processor 50 determines at step 162 that the analyte measurement test is not complete, the process 150 advances to step 164 where the UI processor 50 is operable to maintain the user buttons 26 disabled, i.e., to continue to disregard user presses of any of the user buttons 26.

If, at step 162, the UI processor 50 determines that the analyte measurement test is complete, the process 150 advances to step 166 where the UI processor 50 is operable to enable the user buttons 26 such that user presses of any of the user buttons 26 have their normal effect when the remote electronic device 12 is powered up. The process 150 ends following step 166. The process 150 thus results in unlocking the user keys 26 following completion of an analyte measurement test, as defined herein, after detecting insertion of a sample carrier 22 into the carrier port 20.

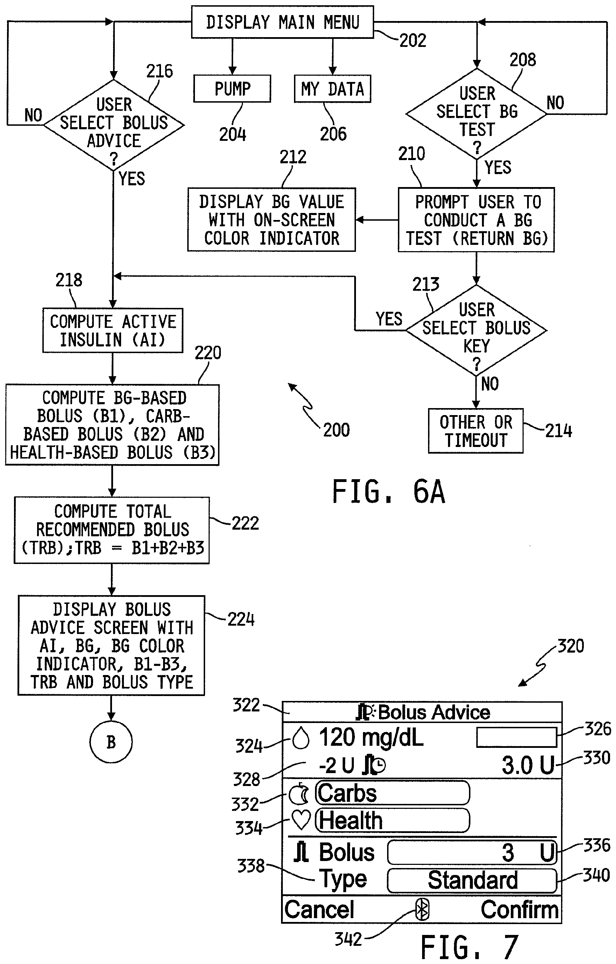

Referring now to FIGS. 6A - 6C, a flow chart is shown of one illustrative embodiment of a bolus advice process 200 that is carried out by the remote electronic device 12. In the context of the flowchart of FIGS. 6A - 6C, the medical device 14 is illustratively implemented in the form of an insulin infusion pump, and will be described as such throughout the description of the process 200. It will be understood, however, that this disclosure contemplates alternate embodiments in which the medical device 14 is or includes other conventional medical devices.

Illustratively, the process 200 is stored in the memory device 66 of the UI processor 50 in the form of instructions that are executable by the UI processor 50 to carry out the bolus advice process 200. The process 200 presumes that the remote electronic device 12 is powered up and that the UI processor 50 is currently controlling the display device 18 to display a main menu 202 that is generally displayed upon power up of the remote electronic device 12. Illustratively, the main menu 202 provides for a number of selectable options that include, but that should not be limited to, a blood glucose (BG) test, a bolus advice process, a pump remote control process 204, a "my data" process 206 and a settings or device set up process.

The pump remote control process 204 provides a menu-driven process by which the remote electronic device 12 may control operation of the insulin infusion pump 14.

In one illustrative embodiment, the "my data" process 206 that is available via the main menu 202 provides for the viewing and editing of diary records, e.g., specific BG test records and pump history records, and also for the analysis of the records over daily and/or weekly time periods. Illustratively, the UI processor 50 stores in the memory 66 up to 1,000 diary records, and up to 250 records may be reviewed using the remote electronic device. The diary records may also be downloaded to a PC or other computer, and using compatible software all records may be viewed and/or analyzed. Each diary record may contain date and time, BG test result, meal time events, carbohydrate value, health event, bolus type and bolus amount. The UI processor can filter and/or sort data from these data records.

The "my data" process also provides for the analysis of the data records in the form of daily and weekly averages, and standard deviations, defined by time slot, and for trend analysis of any of the collected data. Standard day and standard week tables or graphs may be generated to view averages and/or trends. Various charting and table options are also available for presenting data in desired formats.

Referring again to FIG. 6A, the BG test process that may be selected from the main menu 202 begins at step 208 where the UI processor 50 determines whether the user has selected the BG test process from the main menu 202. If not, the "NO" branch of step 208 loops back to the beginning of step 208. If, at step 208, the UI processor 50 determines that the user has selected the BG process from the main menu 202, the process 200 advances to step 210 where the UI processor 50 controls the display device 18 to prompt a user to conduct a BG test. In one embodiment, the UI processor 50 controls the display device 18 to visually guide a user through a blood glucose measurement sequence in which a user inserts a carrier 22 into the glucose measurement facility 20 of the remote electronic device 12 and deposits a sample of blood on the carrier 22, after which the blood glucose meter 88 analyzes the blood sample in a conventional manner to produce a blood glucose (BG) value that corresponds to a concentration of glucose in the deposited blood sample. The blood glucose value, BG, is provided by the blood glucose meter 88 that is on-board the remote electronic device 12 to the UI processor 50 as describe hereinabove. From step 210, the process 300 advances to step 212 where the UI processor 50 is illustratively operable to control the display device 18 to display the BG value along with an on-screen color indicator that is based on the BG value relative to one or more reference BG values.

In the illustrated embodiment, the BG value display screen provides a bolus option that a user may select to enter the bolus advice process directly from the BG measurement process. The UI processor 50 is accordingly operable following step 210, to determine at step 213 whether the user has selected the bolus key from the BG measurement screen. If not, the process 200 advances to step 214 where the user has pressed another key or has selected another option, or alternatively has done nothing and caused the BG value screen to time out. Generally, the UI processor 50 is operable to store the BG value in the memory unit 66 along with measurement time and date information. Illustratively, the user may also store additional information along with the time and date stamp BG value, examples of which may include, but should not be limited to, the timing of the BG measurement relative to meal, bedtime and/or awake time information, amount of carbohydrates taken at the time of the BG measurement, health information such as exercise level, illness or stress, and the like. In any case, if the UI processor 50 determines at step 213 that the user has selected the bolus key option from the BG value display screen, the process 200 advances to step 218.

From the main menu 202, the user may alternatively select the bolus advice process, and the UI processor 50 is accordingly operable at step 216 to determine if the user has selected the bolus advice process. If not, the "NO" branch step 216 loops back at the beginning of step 216, and otherwise the process 200 advances to step 218 where the UI processor 50 is operable to compute an active insulin value, AI, which corresponds to an amount of insulin taken by the user that is currently active. Thereafter at step 220, the UI processor 50 is operable to compute a first bolus value, B1 that is illustratively based on a recent blood glucose value and also on pump operating history data. In embodiments in which blood glucose has not recently been measured, B1 = 0. Also at step 220, the UI processor 50 is operable to compute a second bolus value, B2, that is illustratively based on CARB values entered by the user that correspond to carbohydrates that the user has taken or is planning to take. Further at step 220, the UI processor 50 is operable to compute a third bolus value, B3 that is illustratively based on health information entered by the user that corresponds to a current health state of the user. As one illustrative example, the health state of the user may correspond to exercise, stress, illness, or the like.

The process 200 advances from step 220 to step 222 where the UI processor 50 is operable to compute a total recommended bolus, TRB, as a sum of B1 - B3. Thereafter at step 224, the UI processor 50 is operable to control the display unit 18 to display a bolus advice screen that shows the active insulin value, AI, any recent blood glucose value, BG, a BG color indicator as described with respect to step 212, B1 - B3, TRB and a bolus type, e.g., standard (STD), multi-wave (MW), extended (EXT), each of which may be used to automatically program the pump 14 from the remote electronic device 12, and two manual types. In one embodiment, if a blood glucose measurement, BG, has been conducted within a predefined time period prior to executing step 224, the UI processor 50 is operable at step 224 to display the bolus advice screen showing the measured blood glucose value, BG. Otherwise, the UI processor 50 may be illustratively operable at step 224 to display "bG Test" on the screen where a BG value would be shown if a current BG value was available. From step 224, the process 200 advances to a sub-process B. In general, any value that was measured by or entered into the remote electronic device 12 or medical device 14 may illustratively be displayed when a screen that includes such a value is displayed if the value was measured or entered within a predefined time period since measuring or entering the value.

Referring now to FIG. 7, a graphic example is shown of one illustrative embodiment of a bolus advice display screen 320 produced by the process 200 of FIG. 6A at the point just prior to executing sub-process B, i.e., just after step 224. In the illustrated example, a Bolus Advice label 322 appears at the top of the screen 320 to indicate that the user is executing the bolus advice feature. A blood drop symbol appears next to the displayed blood glucose value 324, e.g., 120 mg/dl, and a color bar 326, providing a visual indication of the blood glucose value 324 relative to an acceptable blood glucose range and/or a number of blood glucose limits, is positioned next to the blood glucose value 324. The active insulin value 328, e.g., - 2U, is positioned under the blood glucose value 324, and a bolus value 330, corresponding to the bolus value B1, is displayed adjacent to the active insulin value 328.

An apple symbol is used to identify a carbohydrates field 332, and a heart symbol is used to identify a health field 334. A bolus type indicator 338 appears below the health field 334 and the total recommended bolus value 3336, e.g., 3 U, is displayed adjacent to the bolus type indicator 338. A bolus type 340, e.g., Standard, is displayed below the total recommended bolus 336. At the bottom of the screen between Cancel and Confirm inputs, a BlueTooth® symbol 342 is provided to indicate the connection status of the wireless communication link with the insulin infusion pump 14, e.g., solid when a wireless connection exists and otherwise flashing.

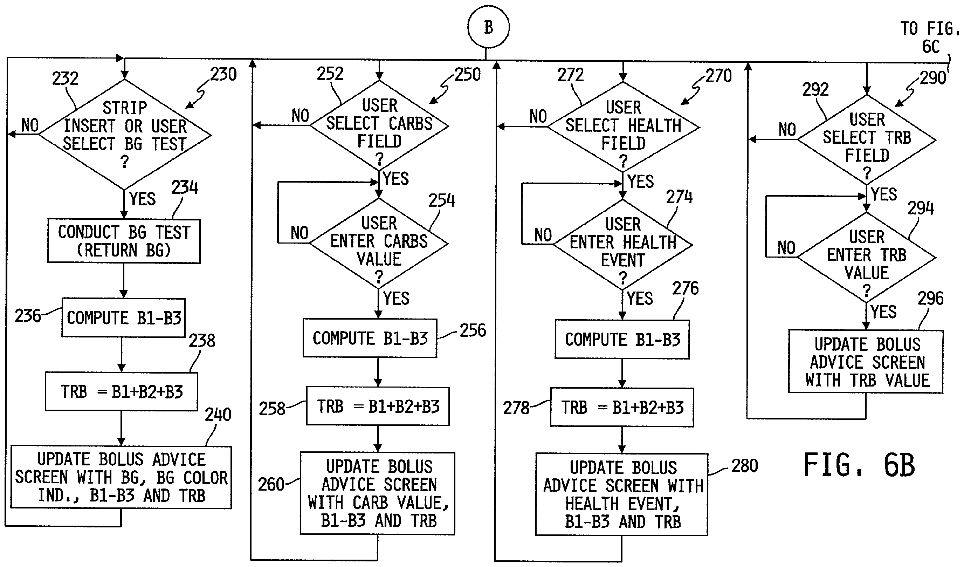

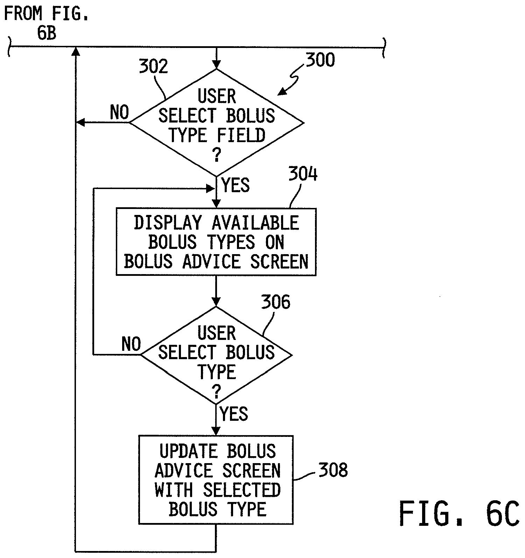

Referring now to FIGS. 6B and 6C, the sub-process B identified after step 224 of FIG. 6A, is shown, wherein the sub-process B forms part of the bolus advice process 200. It will be observed that the sub-process B includes a number of processes that may each be accessed independently any number of times. For example, the sub-process B includes a process 230 for measuring a blood glucose value and computing a bolus amount, B1 that is based, at least in part, on the BG measurement. In the illustrated embodiment, the process 230 begins at step 232 where the UI processor 50 is operable to determine whether a strip insert, e.g., insertion of a blood glucose strip into the carrier port 20 of the remote electronic device 12, has been detected or if the user has selected the bG Test field if this field is displayed in place of a blood glucose value as described above. If not, the process 230 loops back to the beginning of step 232. If, at step 232, the UI processor 50 determines that a strip insert or user selection of the bG Test field has been detected, the process 400 advances to step 234 where the UI processor 50 is operable to conduct a blood glucose test, e.g., by prompting and guiding a user through such a BG test as described above, which returns a measured blood glucose value, BG. Thereafter at step 236, the UI processor 50 is operable to compute all of the bolus values, B1 - B3. In the illustrated embodiment, measured and/or user entered values may have an effect on one or more of the bolus values, B1 - B3, and the UI processor 50 is accordingly operable in the sub-process B to re-compute each bolus value, B1 - B3, after each BG measurement, Carbohydrate entry or health entry. In any case, following step 236 the UI processor 50 is operable at step 238 to compute a total recommended bolus value, TRB, as a sum of B1 and two other bolus values, B2 and B3. Thereafter at step 240, the UI processor 50 is operable to update the bolus advice screen with BG, a BG color indicator as described above, B1 - B3 and TRB. From step 240, the process 200 loops back to the beginning of the sub-process B.

The sub-process B of FIG. 6B further includes a process 250 for entering a carbohydrate value of a meal or snack that has just been, or is planned to be taken, and determining a bolus value based on the entered carbohydrate value. The process 250 begins at step 252 where the UI processor 50 is operable to determine whether a user has selected the CARB field of the displayed bolus advice screen, e.g., item 332 illustrated in FIG. 7. If not, the process 250 loops back to the beginning of step 252. If user selection of the CARBS field is detected at step 252, the process 250 advances to step 254 where the processor 50 is operable to determine whether a carbohydrate value relating to a meal or snack that was just, or is planned to be, taken, has been entered by the user. If not, the process 250 loops back to the beginning of step 254. In one embodiment, the user at step 254 may manually enter a carbohydrate value into the remote electronic device 12, e.g., via the user buttons 16, that corresponds to a carbohydrate content of the meal or snack that was just taken or is planned to be taken. If the user has entered a carbohydrate value that is detected by the UI processor 50 at step 254, the process 250 advances to step 256 where the UI processor 50 is operable to re-compute each of the bolus values B1 - B3. Thereafter at step 258, the UI processor 50 is operable to compute the total recommended bolus, TRB, as the sum of B1 - B3. Thereafter at step 260, the UI processor 50 is operable to control the display device 18 to update the bolus advice display screen to include the carbohydrate value provided by the user at step 224 to display the computed bolus values, B1 - B3, determined at step 256 and to display the updated total recommended bolus value, TRB. The process 250 loops from step 260 back to the beginning of the sub-process B.

The sub-process B of FIG. 6B further includes a process 270 for entering a health information, and determining a bolus value based on the entered health information. The process 270 begins at step 272 where the UI processor 50 is operable to determine whether a user has selected the Health field of the displayed bolus advice screen, e.g., item 334 illustrated in FIG. 7. If not, the process 270 loops back to the beginning of step 272. If user selection of the Health field is detected at step 272, the process 270 advances to step 274 where the processor 50 is operable to determine whether a Health value has been entered by the user. If not, the process 270 loops back to the beginning of step 274. In one embodiment, when the user manually selects at step 272 the health event field displayed on the display device 18 by the UI processor 50, the UI processor 50 is operable to control the display device 18 to display a plurality of health event choices. The health event choices may include, for example, but should not be limited to, no entry, one or more exercise options, an illness option and a sickness option, although more, fewer and/or different options may alternatively be available. In this embodiment, the user may define percentage values associated with each of the health event options during the device set up process such that when the user manually selects one of the health event options at step 274, the UI processor 50 is operable thereafter at step 276 to re-compute the bolus values B1 - B3. Thereafter at step 278, the UI processor 50 is operable to again compute the total recommended bolus, TRB, e.g., as a sum of the individual bolus values, B1-B3. The process 270 advances from step 278 to step 280 where the UI processor 50 is operable to control the display device 18 to update the bolus advice display to include the health event, the bolus values, B1 - B3, and the total recommended bolus value, TRB. The process 270 loops from step 270 back to the beginning of the sub-process B.

The sub-process B of FIG. 6B further includes a process 290 that allows the user to manually modify the total recommended bolus value, TRB. The process 290 begins at step 292 where the UI processor 50 is operable to determine whether a user has selected the TRB field of the displayed bolus advice screen, e.g., item 336 illustrated in FIG. 7. If not, the process 290 loops back to the beginning of step 292. If user selection of the TRB field is detected at step 292, the process 290 advances to step 294 where the processor 50 is operable to determine whether the user has modified the TRB value. If not, the process 290 loops back to the beginning of step 294. If the processor 50 determines that the user has, at step 294, modified the TRB value, the process 290 advances to step 296 where the UI processor 50 is operable to control the display device 18 to update the bolus advice display to include the modified total recommended bolus value, TRB. The process 290 loops from step 296 back to the beginning of the sub-process B.

Referring now to FIG. 6C, the sub-process B further includes a process 300 for selecting a bolus type. The process 300 begins at step 302 where the UI processor 50 is operable to determine whether a user has selected the bolus type field of the displayed bolus advice screen, e.g., item 340 illustrated in FIG. 7. If not, the process 300 loops back to the beginning of step 302. If user selection of the bolus type field is detected at step 302, the process 300 advances to step 304 where the processor 50 is illustratively operable to display available bolus types on the bolus advice screen. In one illustrative embodiment, the available bolus types may include, but should not be limited to, a standard bolus, (STD), a multi-wave (MW) bolus, an extended (EXT) bolus, a manually programmable bolus and a manually administered bolus via an insulin pen or syringe or the like. In cases were a wireless connection between the remote electronic device 12 and 14 is or can be established, the available bolus types may illustratively include all bolus types that the pump 14 is currently capable of delivering. In other cases where a wireless connection could not be established when first entering the bolus advice process, and cannot currently be established, the available bolus types may illustratively include only manually programmable and/or manual delivery via insulin pen/syringe. Those skilled in the art will recognize more, fewer and/or different bolus types that may be made available to the user at step 304, and any such alternative or additional bolus types are contemplated by this disclosure.

Following step 304, the process 300 advances to step 306 where the UI processor 50 is operable to determine whether the user has selected a bolus type. If not, the process 300 loops back to step 304. If the processor 50 determines that the user has, at step 306, selected a bolus type, the process 300 advances to step 306 where the UI processor 50 is operable to control the display device 18 to update the bolus advice display to include the selected bolus type. The process 300 loops from step 308 back to the beginning of the sub-process B.

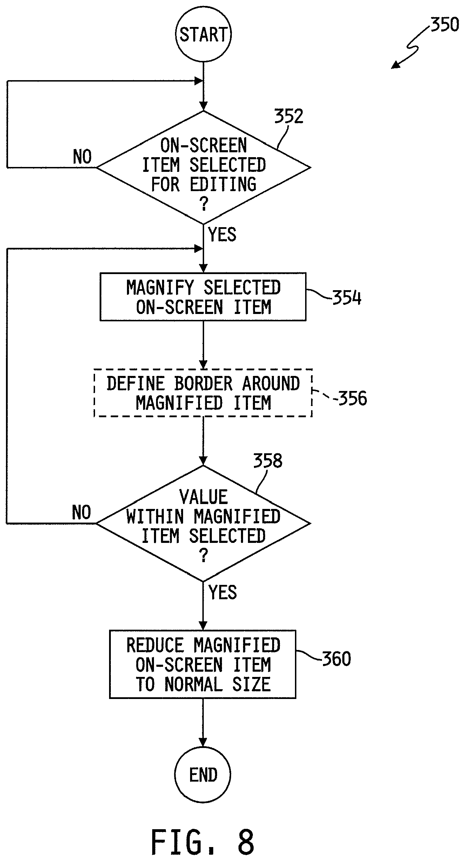

Illustratively, the UI processor 50 may be configured to provide for an expanded editing screen in any situation, such as, for example, but not limited to the processes 250 and 270 of FIG. 6B, in which the user is requested to enter information into the remote electronic device 12 or in which the user enters information into the remote electronic device pursuant to a device set up process. Referring to FIG. 8, a flowchart is shown of one illustrative embodiment of a process 350 by which the UI processor 50 may control the display device 18 to provide for such an expanded editing screen. The process 350 is illustratively stored in the memory unit 66 in the form of instructions that are executable by the UI processor 50 to carry out the process 350. It will be understood that the process 350 may alternatively or additionally be stored in the memory 29 of the processor 28 of the insulin infusion pump 14 in the form of instructions that are executable by the processor 28 to provide for an expanded editing screen when entering data, commands or the like into the insulin infusion pump 14.

The process 350 begins at step 352 where the UI processor 50 determines whether an on-screen item has been selected for editing. Illustratively, the user typically selects an on-screen item for editing by navigating to a desired on-screen item using the up, down, left or right navigation buttons 124, 126, 128 and 130 respectively, and then selecting the desired on-screen item by pressing the Enter button 122. When an on-screen item is so selected, the process 350 advance to step 354 where the UI processor 50 is operable to control the display device 18 to magnify the selected on-screen item such that it appears with larger text and/or numbers than prior to selection. Optionally at step 356, the UI processor 50 may be further operable to define a border, e.g., an edit box or other polygon, around or about the magnified item. Thereafter at step 358, the UI processor 50 is operable to determine whether a value within the magnified item has been selected, e.g., by user press of the Enter button 122. If not, the process 350 loops back to step 354 where the UI processor 50 continues to control the display device 18 to magnify the selected on-screen item. If the UI processor 50 otherwise determines at step 358 that a value with the magnified item has been selected, the process 350 advances to step 360 where the UI processor 50 is operable to reduce the magnified on-screen item to normal size, e.g., the size of the selected item prior to step 354. Following step 360, the process 350 ends.

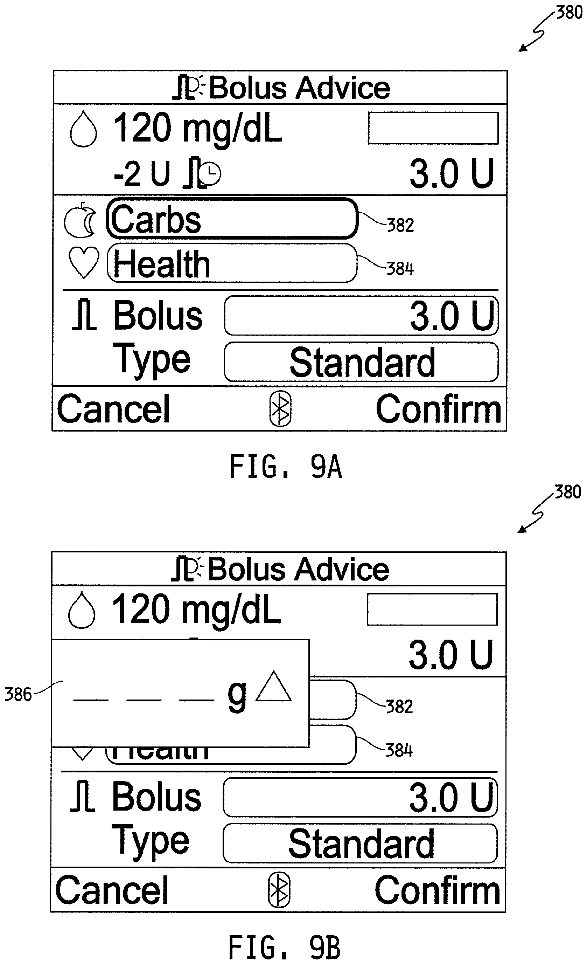

Referring now to FIGS. 9A-9H, graphical representations of one illustrative embodiment of a bolus advice display screen 380 are shown demonstrating the use of expanding user edit areas on the display device 18 according to the process 350 of FIG. 8. FIG. 9A illustratively represents an example state of the display screen 380 at the beginning of step 252 of the process 250 illustrated in FIG. 6B. Here, the user has navigated to the carbohydrate field 382, as is indicated by the bold line circumscribing the carbohydrate field 382, and the carbohydrate field 382 is monitored by the UI processor 50 to determine whether the user selects the carbohydrate field 382 for editing. The bold outline about the carbohydrate field in FIG. 9A may illustratively represent an actual bold outline of the on-screen CARBS data item or field 382, or may alternatively represent some other conventional highlighting technique for drawing the user's attention to the CARBS item or field. In any case, FIG. 9B represents an example state of the display screen 380 when the user selects the CARB item or field 382, e.g., by pressing the Enter button 122 when the CARBS item or field 382 is highlighted.

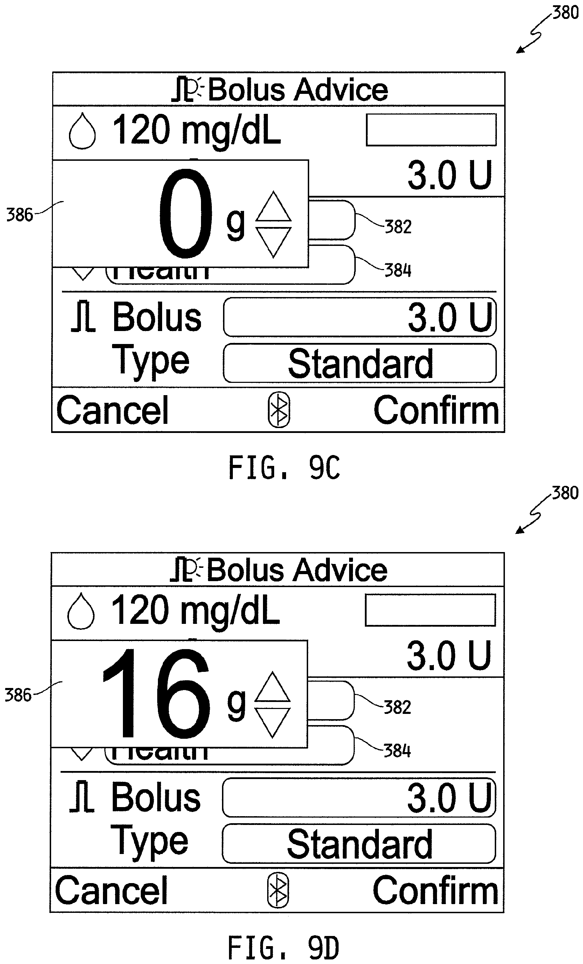

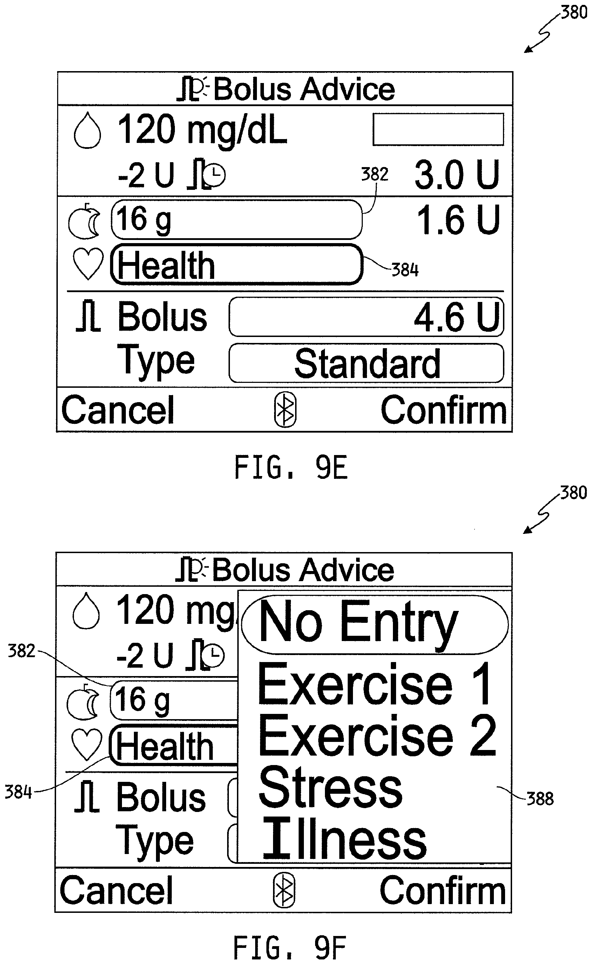

When the user selects the CARB item or field, such as is illustrated in FIG. 9B, the UI processor 50 controls the display device 18 to produce an expanded edit area 386 that magnifies the selected CARB item or field 382 relative to other portions of the display device 18 while also providing a magnified field that the user may edit. In one embodiment, as illustrated in FIGS. 9A-9H, the expanded edit field is illustratively accompanied by a unit of measure, e.g., carbohydrates are illustrated in units of grams. In the illustrated embodiment, the expanded edit area 386 is surrounded by a border in the form of, for example, a solid-colored rectangle. When the user presses the up button 124, as indicated in the expanded edit area 386 by the up arrow, the UI processor 50 modifies the display device 18 to produce a zero in the expanded edit area 386 along with an up arrow and a down arrow as shown in FIG. 9C. The user may then select the up button 124 or the down button 126 to increase and decrease the displayed value. Illustratively, the UI processor may implement a fast scroll algorithm that allows the CARBS value to change rapidly and/or to increase the increment value, e.g., in blocks of 5, 10 or other number, when the up button 124 or the down button 126 is pressed and held. As shown in FIG. 9D, the user has repeatedly pressed the up button 124 to indicate that the meal or snack comprises 16 grams of carbohydrates. The user then presses the Enter button 122 to enter the 16 gram selection in the CARBS field, and when this is done the UI processor 50 is operable to control the display device 18 to reduce the magnified on-screen CARBS field 386 back to its default size as shown in FIG. 9E.

As also illustrated in FIG. 9E, the UI processor 50 has computed a bolus, (1.6U), from the entered CARBS value and updated the bolus advice display 380 according to steps 256 - 260 of the process 250 of FIG. 6B. In this example, B2 is computed according to the equation B2 = CARBS*CR, where CR is a carbohydrate ratio value that the user has defined in a setup screen as 1U/10g so that B2 = 1.6. The UI processor 50 has also updated the total recommended bolus field to automatically add the B1 bolus value and the B2 bolus value for a total of 4.6 U of insulin.

As further illustrated in FIG. 9E, when the user enters a carbohydrate value into the CARBS field 382, the UI processor 50 automatically prompts the user to enter health event information as represented in FIG. 9E by the bold outline around the Health field 384 of the example bolus advice screen 380. The bold outline may illustratively represent an actual bold outline of the on-screen Health item or field 384, or may alternatively represent some other conventional highlighting technique for drawing the user's attention to the Health item or field 384. In any case, FIG. 9F represents an example state of the display screen 380 when the user selects the Health item or field 384, e.g., by pressing the Enter button 122 when the Health item or field 384 is highlighted.

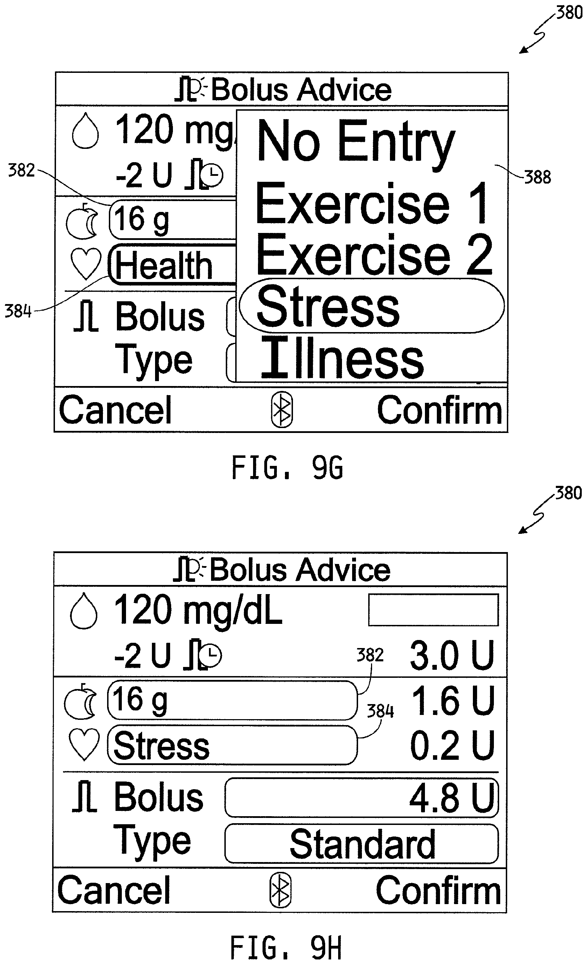

When the user selects the Health item or data field, the UI processor 50 controls the display device 18 to produce an expanded edit area 388 that magnifies the selected Health item or data field 384 while also providing a number of selectable health options. In the illustrated embodiment, the selectable health options include No Entry, Exercise 1, Exercise 2, Stress and Illness, although this list may alternatively include more, fewer and/or different health-related options. Illustratively, the NO Entry item is the default item in the Health list 388, and is therefore highlighted as represented by the outline around No Entry. The user may use the up and down buttons 124, 126 respectively to navigate the list displayed in the expanded edit area 388, and may select a desired one of the health items on the list by pressing the Enter button 122 when the desired health item is highlighted. In the example illustrated in FIGS. 9G and 9H, the user has chosen and selected Stress as the health item in the expanded edit area 388. When the user presses the Enter button 122 to enter the Stress item in the Health field, the UI processor 50 is operable, as shown in FIG. 9H, to control the display device 18 to reduce the magnified on-screen item 384 back to its default size.

In one embodiment, the UI processor 50 may be operable to deactivate the confirm function illustrated in FIGS. 9A - 9H at the bottom right corner of the screen 380. In this embodiment, a bolus cannot be confirmed which the user is editing certain functions of the bolus advice process. In alternative embodiments, the UI processor 50 may control the display 18 such that the confirm function is taken away, i.e., is not viewable, during the illustrated editing process. In other alternative embodiments, the confirm function may be fully operational during the illustrated editing process.

As also illustrated in FIG. 9H, the UI processor 50 has computed a bolus, B3, from the entered Health item and updated the bolus advice display 380 according to steps 276 and 280 of the process 270 of FIG. 6B. In this example, B3 is computed according to the equation B3 = Stress%*(B1 + B2, where Stress% is a percentage value that the user has defined in a setup screen as 5% so that B3 = 0.23 = 0.2. The UI processor 50 has also updated the total recommended bolus field to automatically add the B1 bolus value and the B2 bolus value and the B3 bolus value, in accordance with step 278 of the process 270 of FIG. 6B, for a total of 4.8 U of insulin.

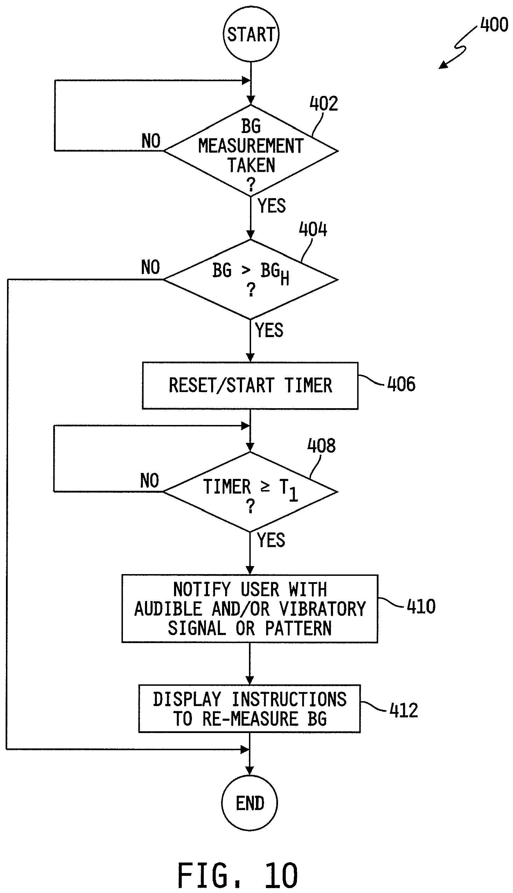

Illustratively, the UI processor 50 is programmed to automatically notify a user via the audible indicator 72 and/or the vibrator device 74 and to display a message to the user via the display device 18 to measure blood glucose if certain measured and/or user entered parameters fall outside one or more ranges or limits. Referring now to FIG. 10, a flowchart is shown of one illustrative embodiment of one such process 400 for automatically notifying and instructing a user to measure blood glucose. Illustratively, the process 400 is stored in the memory unit 66 in the form of instructions that are executable by the UI processor 50 to automatically notify and instruct a user to measure blood glucose. The process 400 begins at step 402 where the UI processor 50 is operable to determine whether a BG measurement has just been taken via the on-board glucose meter 88. Generally, the UI processor 50 will be notified when a BG measurement has been taken by the ME processor 56 when the ME processor provides a measured blood glucose value to the UI processor 50 via the TXD line. If the UI processor 50 determines at step 402 that a BG measurement has not been taken, the process 400 loops back to the beginning of step 402. Otherwise, the process 400 advances to step 404 where the UI processor 50 is operable to determine whether the BG value just measured (BG) is greater than a high BG value, BG_{H}. Illustratively, BG_{H} may be a hyperglycemic threshold value, although BG_{H} may alternatively be a different high blood glucose value.

If, at step 404, the UI processor 50 determines that the BG measurement just taken is greater than BG_{H}, the process 400 advances to step 406 where the UI processor 50 is operable to reset and start an internal timer. Thereafter at step 408, the UI processor 50 is operable to determine whether the count value of the internal timer has reached a time value, T1, e.g., is greater than or equal to T1. In one embodiment, the time value T1 may be selected by the user in a set up menu. Alternatively, the time value T1 may be set by a health care professional or may be set during manufacture, and in either case may not be modified by the user. If, at step 408, the UI processor 50 determines that the count value of the timer is not greater than or equal to T1, the process 400 loops back to the start of step 408. When the count value of the timer reaches T1, the process 400 advances to step 410 where the UI processor 50 is operable to notify the user with an audible and/or vibratory signal or pattern of signals. Thereafter at step 412, the UI processor 50 is operable to control the display device 18 to display instructions to the user to re-measure blood glucose. From step 412, and from the "NO" branch of step 404, the process 400 ends.

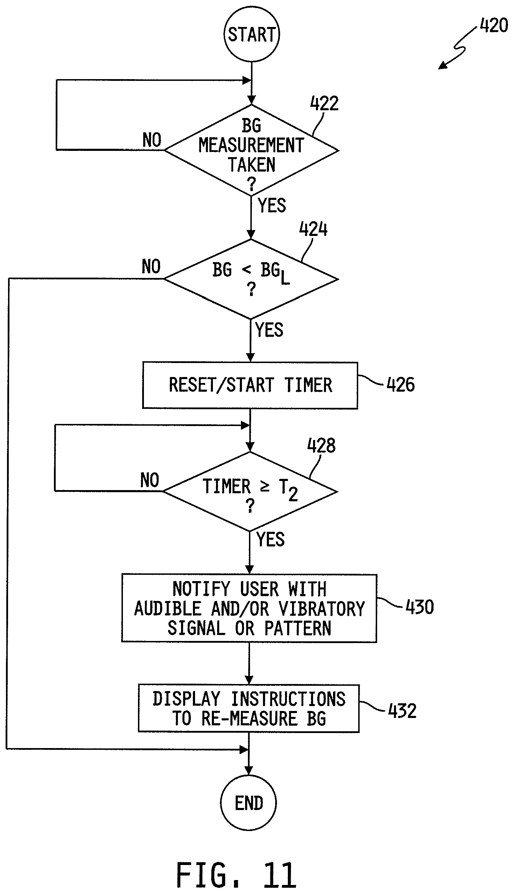

Referring now to FIG. 11, a flowchart is shown of another illustrative embodiment of a process 420 for automatically notifying and instructing a user to measure blood glucose. Illustratively, the process 420 is stored in the memory unit 66 in the form of instructions that are executable by the UI processor 50 to automatically notify and instruct a user to measure blood glucose. The process 420 begins at step 422 where the UI processor 50 is operable to determine, e.g. as described above, whether a BG measurement has just been taken via the on-board glucose meter 88. If the UI processor 50 determines at step 422 that a BG measurement has not been taken, the process 420 loops back to the beginning of step 422. Otherwise, the process 420 advances to step 424 where the UI processor 50 is operable to determine whether the BG value just measured (BG) is less than a low BG value, BG_{L}. Illustratively, BG_{L} may be a hypoglycemic threshold value, although BG_{L} may alternatively be a different low blood glucose value.

If, at step 424, the UI processor 50 determines that the BG measurement just taken is less than BG_{L}, the process 420 advances to step 426 where the UI processor 50 is operable to reset and start an internal timer. Thereafter at step 428, the UI processor 50 is operable to determine whether the count value of the internal timer has reached, e.g., is greater than or equal to, a time value, T2. In one embodiment, the time value T2 may be selected by the user in a set up menu. Alternatively, the time value T2 may be set by a health care professional or may be set during manufacture, and in either case may not be modified by the user. If, at step 428, the UI processor 50 determines that the count value of the timer is not greater than or equal to T2, the process 420 loops back to the start of step 428. When the count value of the timer reaches T2, the process 420 advances to step 430 where the UI processor 50 is operable to notify the user with an audible and/or vibratory signal or pattern of signals. Thereafter at step 432, the UI processor 50 is operable to control the display device 18 to display instructions to the user to re-measure blood glucose. From step 432, and from the "NO" branch of step 424, the process 420 ends.

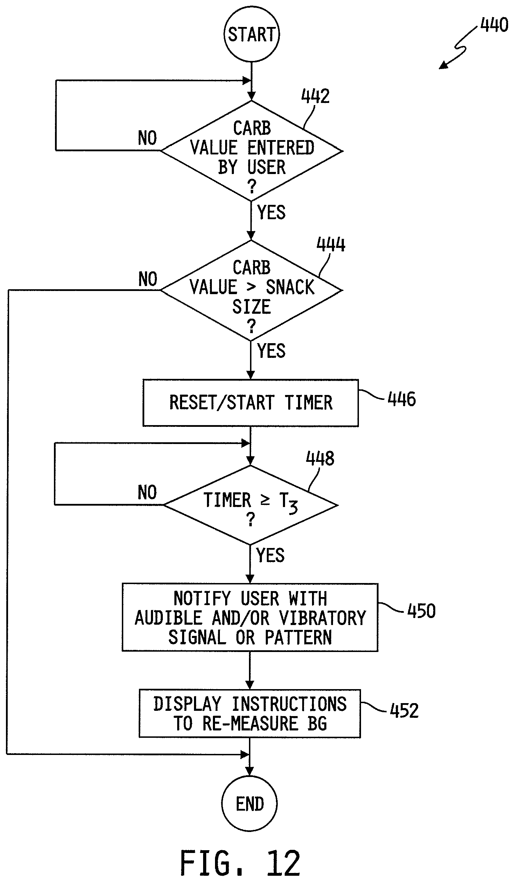

Referring now to FIG. 12, a flowchart is shown of yet another illustrative embodiment of a process 440 for automatically notifying and instructing a user to measure blood glucose. Illustratively, the process 440 is stored in the memory unit 66 in the form of instructions that are executable by the UI processor 50 to automatically notify and instruct a user to measure blood glucose. The process 440 begins at step 442 where the UI processor 50 is operable to determine whether a carbohydrate value has been entered by a user, e.g., using the bolus advice process. If so, the process 440 advances to step 444 where the UI processor 50 is operable to determine whether the carbohydrate value entered at step 442 is larger than a predetermined, e.g., programmable, snack size. If so, the process 440 advances to step 446 where the UI processor 50 is operable to reset and start an internal timer. Thereafter at step 448, the UI processor 50 is operable to determine whether the count value of the internal timer has reached a time value, T3. In one embodiment, the time value T3 may be selected by the user in a set up menu. Alternatively, the time value T3 may be set by a health care professional or may be set during manufacture, and in either case may not be modified by the user. If, at step 448, the UI processor 50 determines that the count value of the timer is not greater than or equal to T3, the process 440 loops back to the start of step 442. When the count value of the timer reaches T2, the process 440 advances to step 450 where the UI processor 50 is operable to notify the user with an audible and/or vibratory signal or pattern of signals. Thereafter at step 452, the UI processor 50 is operable to control the display device 18 to display instructions to the user to measure blood glucose. From step 452, and from the "NO" branch of step 444, the process 440 ends.

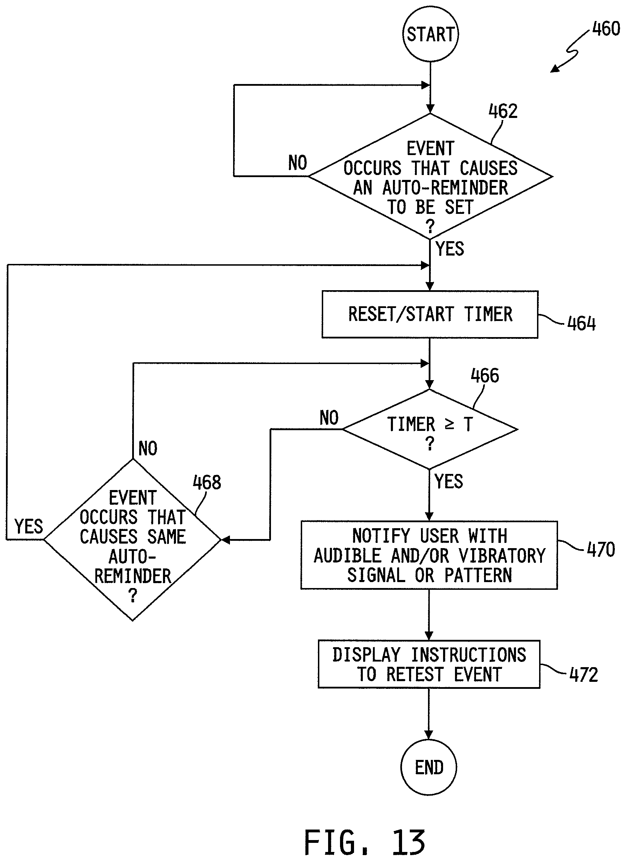

Referring now to FIG. 13, a flowchart is shown of one illustrative embodiment of a process 460 for canceling an automatic notification upon reoccurrence of an event that caused the notification to be programmed. Illustratively, the process 460 is stored in the memory unit 66 in the form of instructions that are executable by the UI processor 50 to selectively cancel automatic notifications under certain conditions. The process 460 begins at step 462 where the UI processor 50 is operable to determine whether an event has occurred that has caused an automatic reminder, e.g., a programmed notification, to be set, i.e., to be programmed in the UI. If so, the process 460 advances to step 464 where the UI processor 50 is operable to reset and start an internal timer. Thereafter at step 466, the UI processor 50 is operable to determine whether the count value of the internal timer has exceeded a time value, T. If not the process 460 advances to step 468 where the UI processor 50 is operable to determine whether the event has occurred that will cause the same auto-reminder, e.g., programmed notification, that is already set or pending. If so, the process 460 advances to step 464 where the timer is reset and started again, thereby canceling the earlier programmed notification in favor of the latter occurring one. If, at step 468, the UI processor 50 determines that an event has not occurred that would cause the same auto-reminder, e.g., programmed notification, to be set, the process 460 advances to step 466. When the count value of the timer reaches T, the process 460 advances to step 470 where the UI processor 50 is operable to notify the user with an audible and/or vibratory signal or pattern of signals. Thereafter at step 472, the UI processor 50 is operable to control the display device 18 to display instructions to the user to retest the event that caused the auto-reminder to be set. From step 472, the process 460 ends.

It should be apparent that the process 460 provides for only one active alarm of one type at a time. As an example, a user measures low blood glucose, and an automatic reminder is automatically set by the low glucose value to notify the user to test blood glucose in thirty minutes. If the user then retests blood glucose twenty minutes later and finds the same low blood glucose value again, the process 460 may cancel the first auto-reminder in favor of the second.

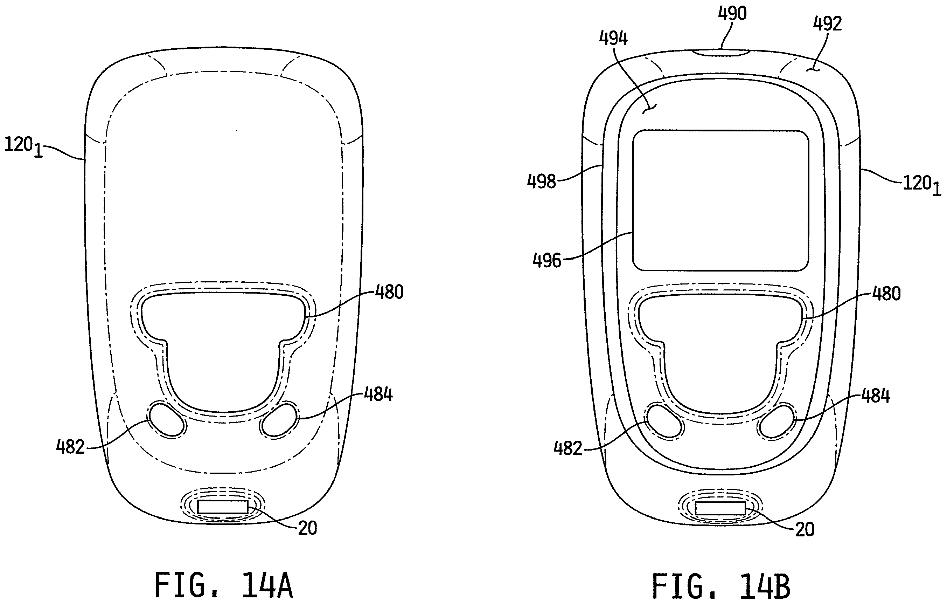

Referring now to FIGS. 14A and 14B, diagrams are shown of one illustrative embodiment of the top portion 120₁ of the housing 120 of the remote electronic device (see FIGS. 1-4). In the illustrated embodiment, the top portion 120₁ of the housing 120 is a single, unitary piece that is illustratively formed of a conventional polymer material, such as via a conventional injection molding process, although this disclosure contemplates using other conventional materials and/or other conventional processes to form the housing top 120₁. In the embodiment illustrated in FIGS. 14A and 14B, the housing top 120₁ is illustratively formed to define through the housing top 120₁ an opening to the carrier port 20, an opening 480 that is sized and shaped to receive the user buttons 16, an opening 482 that is sized and shaped to receive the on/off button 136 and an opening 484 that is sized and shaped to receive the backlight button 138. Although not specifically shown in FIGS. 14A and 14B, the user buttons 16 extend into and at least partially through the opening 480 when the user buttons 16 are positioned within the housing 120, and the buttons 136 and 138 likewise extend into and at least partially through the openings 482 and 484 respectively when the buttons 136 and 138 are received within the housing 120.

The housing top 120₁ illustrated in FIG. 14A is illustratively formed of a light transmissive polymer. In one embodiment, the housing top 120₁ is formed of a transparent polymer, i.e., such that it transmits light without appreciable scattering so that objects beyond are seen clearly. In this embodiment, the housing top 120₁ may be clear or may alternatively be colored, e.g., tinted. In alternative embodiments, the housing top 120₁ may be at least partially translucent. In any case, the housing top 120₁ is further processed after its initial formation to define a number of integral windows through the housing top 120₁.

In the example embodiment illustrated in FIG. 14B, the housing top 120₁ is further processed to define two such integral windows 490 and 496. In this example, the integral window 490 defined by the housing top 120₁ is approximately D-shaped, although the window 49 may alternatively be formed in any desired shape. In any case, the window 490 is positioned relative to the housing 120 and relative to the electrical circuitry carried by the housing 120 to extend over and adjacent to the IR transceiver 65 so that the transceiver 65 has a line of sight through the housing 120. Another integral window 496 defined by the housing top 120₁ is generally rectangular in shape, although the window 496 may alternatively be formed in any desired shape. The integral window 496 is illustratively positioned relative to the housing 120 and relative to the electrical circuitry carried by the housing 120 to extend over a viewing area of the display unit 18 so that the display unit 18 can be viewed through the integral window 496.

In the illustrated embodiment, the integral windows 490 and 496 are defined by coating the outer surface of the top housing 120₁ with an opaque or other non-light transmissive coating while suitably masking the window areas 490 and 496 from the coating. In one example embodiment, the outer surface of the top housing 120₁, with the exception of the windows 490 and 496, are painted with a suitable acrylic or oil-based paint, although alternative and/or additional coatings or coating types are contemplated by this disclosure. In the example embodiment illustrated in FIG. 14B, three different coating areas are defined. A first coating area 492 is defined around a perimeter of the housing top 120₁, a second coating area 494 is defined on the top portion of the housing top 120₁ and a third coating area 498 is defined in the form of a strip that separates the coating areas 492 and 494. In the illustrated embodiment, the coating areas 492, 494 and 498 represent different colors, although the coating areas 492, 494 and 498 may alternatively or additionally define different shades and/or textures.
WHILE THE INVENTION HAS BEEN ILLUSTRATED AND DESCRIBED IN DETAIL IN THE FOREGOING DRAWINGS AND DESCRIPTION, THE SAME IS TO BE CONSIDERED AS ILLUSTRATIVE AND NOT RESTRICTIVE IN CHARACTER, IT BEING UNDERSTOOD THAT ONLY ILLUSTRATIVE EMBODIMENTS THEREOF HAVE BEEN SHOWN AND DESCRIBED AND THAT THE SCOPE OF THE INVENTION IS DEFINED BY THE APPENDED CLAIMS.

## Claims

1. A method of controlling a display device (18) carried by a housing of a blood glucose measuring device (12), the blood glucose measuring device (12) comprising:
a blood glucose measuring facility (88) positioned in the housing and configured to measure a concentration of glucose in a blood sample deposited on a sample carrier received in the blood glucose measuring facility, and
a processor (50) including a memory having instructions stored therein that are executable by the processor:
to control a bolus recommendation process that recommends a bolus amount of a blood glucose modifying drug based on a number of factors including a carbohydrate value entered by a user, wherein the entered carbohydrate value corresponds to a carbohydrate content of a meal or snack that was just taken, and
to control the display device, after a programmable amount of time has passed since entering the carbohydrate value, to display instructions to measure a concentration of blood glucose of a blood sample via the blood glucose measuring facility if the carbohydrate value entered by the user is greater than a carbohydrate limit,
the method comprising:
displaying a data field on the display device,
executing an editing process that provides for editing of the data field displayed on the display device, and
magnifying the data field on the display device relative to other portions of the display device when selected for editing according to the editing process.

2. The method according to claim 1, **characterized in that** magnifying the data field comprises producing an expanded polygon about the magnified data field.

3. The method according to claim 1 or 2, **characterized in that** the method further comprises:
displaying a plurality of data fields on the display device, and
magnifying any of the plurality of data fields when selected for editing according to the editing process.

4. The method according to any of claims 1 to 3, **characterized in that** the magnified data field is populated with a plurality of selectable choices when selected for editing according to the editing process.

5. The method according to any of claims 1 to 4, **characterized in that** the method further comprises incrementally increasing a value displayed within the magnified data field by an increment value in response to press of a user button of the blood glucose measuring device.

6. The method according to claim 5, **characterized in that** the method further comprises a fast scroll process that incrementally increases the value displayed within the magnified field at a rapid rate when the user button is pressed and held.

7. The method according to claim 5 or 6, **characterized in that** the method further comprises a fast scroll process that increases the increment value when the user button is pressed and held.

8. The method according to any of claims 1 to 7, **characterized in that** the method further comprises incrementally decreasing a value displayed within the magnified data field in response to press of a user button of the blood glucose measuring device.

9. The method according to claim 8, **characterized in that** the method further comprises a fast scroll process that incrementally decreases the value displayed within the magnified field at a rapid rate when the user button is pressed and held.

10. The method according to claim 8 or 9, **characterized in that** the method further comprises a fast scroll process that increases the decrement value when the user button is pressed and held.

11. The method according to any of claims 1 to 10, **characterized in that** the method further comprises automatically displaying a unit of measure of data within, or to be entered within, the magnified data field.

## Patentansprüche

1. Verfahren zum Steuern einer Anzeigevorrichtung (18), die von einem Gehäuse einer Blutzuckermessvorrichtung (12) getragen wird, wobei die Blutzuckermessvorrichtung (12) umfasst:
eine Blutzuckermessvorrichtung (88), die in dem Gehäuse positioniert und konfiguriert ist, um eine Glukosekonzentration in einer Blutprobe zu messen, die auf einem in der Blutzuckermessvorrichtung aufgenommenen Probenträger aufgebracht ist, und einen Prozessor (50), der einen Speicher mit darin gespeicherten Anweisungen umfasst, die durch den Prozessor ausführbar sind:
um einen Bolus-Empfehlungsprozess zu steuern, der eine Bolusmenge eines blutzuckermodifizierenden Arzneimittels basierend auf einer Anzahl von Faktoren einschließlich eines durch einen Benutzer eingegebenen Kohlenhydratwertes empfiehlt, wobei der eingegebene Kohlenhydratwert einem Kohlenhydratgehalt einer Mahlzeit oder eines Snacks entspricht, die/der gerade zu sich genommen wurde, und
um die Anzeigevorrichtung zu steuern, nachdem eine programmierbare Zeitdauer seit dem Eingeben des Kohlenhydratwerts vergangen ist, um Anweisungen zum Messen einer Konzentration von Blutzucker einer Blutprobe mittels der Blutzuckermessvorrichtung anzuzeigen, wenn der durch den Benutzer eingegebene Kohlenhydratwert größer ist als ein Kohlenhydratgrenzwert,
wobei das Verfahren umfasst:
Anzeigen eines Datenfeldes auf der Anzeigevorrichtung, Ausführen eines Bearbeitungsprozesses, der das Bearbeiten des auf der Anzeigevorrichtung angezeigten Datenfeldes bereitstellt, und
Vergrößern des Datenfeldes auf der Anzeigevorrichtung relativ zu anderen Abschnitten der Anzeigevorrichtung, wenn dies zum Bearbeiten gemäß dem Bearbeitungsprozess ausgewählt wird.

2. Verfahren nach Anspruch 1, **dadurch gekennzeichnet, dass** das Vergrößern des Feldes das Erzeugen eines erweiterten Polygons um das vergrößerte Datenfeld umfasst.

3. Verfahren nach Anspruch 1 oder 2, **dadurch gekennzeichnet, dass** das Verfahren ferner umfasst:
Anzeigen einer Mehrzahl von Datenfeldern auf der Anzeigevorrichtung und Vergrößern eines beliebigen der Mehrzahl von Datenfeldern, wenn es zum Bearbeiten gemäß dem Bearbeitungsprozess ausgewählt wird.

4. Verfahren nach einem der Ansprüche 1 bis 3, **dadurch gekennzeichnet, dass** das vergrößerte Datenfeld mit einer Mehrzahl von auswählbaren Auswahlmöglichkeiten bestückt ist, wenn es zum Bearbeiten gemäß dem Bearbeitungsprozess ausgewählt wird.

5. Verfahren nach einem der Ansprüche 1 bis 4, **dadurch gekennzeichnet, dass** das Verfahren ferner das schrittweise Erhöhen eines in dem vergrößerten Datenfeld angezeigten Werts um einen Inkrementwert als Reaktion auf das Drücken einer Benutzerschaltfläche der Blutzuckermessvorrichtung umfasst.

6. Verfahren nach Anspruch 5, **dadurch gekennzeichnet, dass** das Verfahren ferner einen schnellen Scroll-Prozess umfasst, der den in dem vergrößerten Feld angezeigten Wert mit einer hohen Geschwindigkeit schrittweise erhöht, wenn die Benutzerschaltfläche gedrückt und gehalten wird.

7. Verfahren nach Anspruch 5 oder 6, **dadurch gekennzeichnet, dass** das Verfahren ferner einen schnellen Scroll-Prozess umfasst, der den Inkrementwert erhöht, wenn die Benutzerschaltfläche gedrückt und gehalten wird.

8. Verfahren nach einem der Ansprüche 1 bis 7, **dadurch gekennzeichnet, dass** das Verfahren ferner das schrittweise Erhöhen eines in dem vergrößerten Datenfeld angezeigten Werts um einen Inkrementwert als Reaktion auf das Drücken einer Benutzerschaltfläche der Blutzuckermessvorrichtung umfasst.

9. Verfahren nach Anspruch 8, **dadurch gekennzeichnet, dass** das Verfahren ferner einen schnellen Scroll-Prozess umfasst, der den in dem vergrößerten Feld angezeigten Wert mit einer hohen Geschwindigkeit schrittweise verringert, wenn die Benutzerschaltfläche gedrückt und gehalten wird.

10. Verfahren nach Anspruch 8 oder 9, **dadurch gekennzeichnet, dass** das Verfahren ferner einen schnellen Scroll-Prozess umfasst, der den Dekrementwert erhöht, wenn die Benutzerschaltfläche gedrückt und gehalten wird.

11. Verfahren nach einem der Ansprüche 1 bis 10, **dadurch gekennzeichnet, dass** das Verfahren ferner das automatische Anzeigen einer Maßeinheit von Daten innerhalb des vergrößerten Datenfeldes oder die darin einzugeben sind umfasst.

## Revendications

1. Procédé de commande d'un dispositif d'affichage (18) porté par un boîtier d'un glucomètre (12), le glucomètre (12) comprenant :
un dispositif de mesure de la glycémie (88) positionné dans le boîtier et configuré pour mesurer une concentration de glucose dans un échantillon de sang déposé sur un support d'échantillon reçu dans le dispositif de mesure de la glycémie, et
un processeur (50) incluant une mémoire stockant des instructions qui sont exécutables par le processeur :
pour commander un processus de recommandation de bolus qui recommande une quantité de bolus d'un médicament modifiant la glycémie sur la base d'un nombre de facteurs comprenant une valeur de glucides entrée par un utilisateur, dans lequel la valeur de glucides entrée correspond à une teneur en glucides d'un repas ou d'un encas qui vient d'être pris, et
pour commander au dispositif d'affichage, après l'écoulement d'une quantité programmable de temps depuis l'entrée de la valeur de glucides, d'afficher des instructions de mesure d'une concentration de glucose d'un échantillon de sang via le dispositif de mesure de la glycémie si la valeur de glucides entrée par l'utilisateur est supérieure à une limite de glucides,
le procédé comprenant :
l'affichage d'un champ de données sur le dispositif d'affichage,
l'exécution d'un processus d'édition qui permet l'édition du champ de données affiché sur le dispositif d'affichage, et
l'agrandissement du champ de données sur le dispositif d'affichage relativement aux autres parties du dispositif d'affichage lorsqu'il est sélectionné pour l'édition selon le processus d'édition.

2. Procédé selon la revendication 1, **caractérisé en ce que** l'agrandissement du champ de données comprend la production d'un polygone expansé autour du champ de données agrandi.

3. Procédé selon la revendication 1 ou 2, **caractérisé en ce que** le procédé comprend en outre :
l'affichage d'une pluralité de champs de données sur le dispositif d'affichage, et
l'agrandissement de l'un quelconque de la pluralité de champs de données lorsqu'il est sélectionné pour l'édition selon le processus d'édition.

4. Procédé selon l'une quelconque des revendications 1 à 3, **caractérisé en ce que** le champ de données agrandi est peuplé d'une pluralité de choix sélectionnables lorsqu'il est sélectionné pour l'édition selon le processus d'édition.

5. Procédé selon l'une quelconque des revendications 1 à 4, **caractérisé en ce que** le procédé comprend en outre l'augmentation incrémentielle d'une valeur affichée à l'intérieur du champ de données agrandi d'une valeur incrémentielle en réponse à l'enfoncement d'un bouton utilisateur du glucomètre.

6. Procédé selon la revendication 5, **caractérisé en ce que** le procédé comprend en outre un processus de défilement rapide qui augmente de façon incrémentielle la valeur affichée dans le champ agrandi à une vitesse rapide lorsque le bouton utilisateur est maintenu enfoncé.

7. Procédé selon la revendication 5 ou 6, **caractérisé en ce que** le procédé comprend en outre un processus de défilement rapide qui augmente la valeur d'incrément lorsque le bouton utilisateur est maintenu enfoncé.

8. Procédé selon l'une quelconque des revendications 1 à 7, **caractérisé en ce que** le procédé comprend en outre la réduction incrémentielle d'une valeur affichée à l'intérieur du champ de données agrandi en réponse à l'enfoncement d'un bouton utilisateur du glucomètre.

9. Procédé selon la revendication 8, **caractérisé en ce que** le procédé comprend en outre un processus de défilement rapide qui réduit de façon incrémentielle la valeur affichée à l'intérieur du champ agrandi à une vitesse rapide lorsque le bouton utilisateur est maintenu enfoncé.

10. Procédé selon la revendication 8 ou 9, **caractérisé en ce que** le procédé comprend en outre un processus de défilement rapide qui augmente la valeur de décrément lorsque le bouton utilisateur est maintenu enfoncé.

11. Procédé selon l'une quelconque des revendications 1 à 10, **caractérisé en ce que** le procédé comprend en outre l'affichage automatique d'une unité de mesure de données à l'intérieur, ou à entrer à l'intérieur, du champ de données agrandi.
